# EUROPÄISCHE PATENTSCHRIFT

(11) **EP 0 751 221 B1**
(45) Veröffentlichungstag und Bekanntmachung des Hinweises auf die Patenterteilung: **09.09.1998**
(21) Anmeldenummer: 95109949.8
(22) Anmeldetag: 26.06.1995
(51) Int. Cl.: C12N 15/29, C07K 14/42, C12N 15/62, A61K 38/16, A61K 35/78, A61K 47/48, C07K 19/00, C12Q 1/68, A61K 45/05

(54) **Rekombinantes Mistellektin (rML)**
Recombinant mistletoe lectin (rML)
Lectine recombinante de gui (rML)

(43) Veröffentlichungstag der Anmeldung: 02.01.1997
(60) Teilanmeldung: 98105660.9
(73) Patentinhaber: MADAUS AG Köln, 51109 Köln (DE)
(72) Erfinder: Lentzen, Hans, 51503 Rösrath (DE); Eck, Jürgen, 64646 Heppenheim (DE); Baur, Axel, 40668 Meerbusch-Ossum (DE); Zinke, Holger, 64404 Bickenbach (DE)
(74) Vertreter: VOSSIUS & PARTNER

(56) Entgegenhaltungen:
- DE-A- 4 221 836
- DE-A- 4 341 476
- ANTICANCER RESEARCH 12 (3). 1992. 669-676, GABIUS, H.-M. ET AL. 'The immunomodulatory beta-galactosidase-specific lectin from mistletoe: partial sequence analysis, cell and tissue binding, and impact on intracellular biosignalling of monocytic leukemia cells'
- ARCH IMMUNOL THER EXP 40 (3-4). 1992. 223-227, PAPROCKA, M. ET AL. 'THE ACTIVITY OF TWO IMMUNOTOXINS COMPOSED OF MONOCLONAL ANTIBODY MOAB-16 AND A-CHAIN OF RICIN MOAB-16-RTA OR A-CHAIN OF MISTLETOE LECTIN I MOAB-16-MLIA.'
- INT J IMMUNOPHARMACOL 13 (7). 1991. 1037-1042, TONEVITSKY, A. ET AL. 'IMMUNOTOXIN WITH MISTLETOE LECTIN I A-CHAIN AND RICIN A-CHAIN DIRECTED AGAINST CD5 ANTIGEN OF HUMAN T-LYMPHOCYTES COMPARISON OF EFFICIENCY AND SPECIFICITY.'
- ANTI-CANCER DRUGS 3 (5). 1992. 507-511, DIETRICH, J. ET AL. 'IDENTITY OF THE N-TERMINAL SEQUENCES OF THE THREE A CHAINS OF MISTLETOE VISCUM -ALBUM L. LECTINS HOMOLOGY WITH RICIN-LIKE PLANT TOXINS AND SINGLE-CHAIN RIBOSOME-INHIBITING PROTEINS.'

## Beschreibung

Die Erfindung betrifft Nukleinsäuremoleküle, die Präproproteine codieren, die nach Reifung die biologische Aktivität des Mistellektindimers aufweisen, Vektoren, die diese Nukleinsäuremoleküle enthalten, mit diesen Vektoren transformierte Wirte und Polypeptide bzw. Polypeptiddimere, die von diesen Nukleinsäuremolekülen codiert werden. Die erfindungsgemäßen Polypeptide bzw. Polypeptiddimere sind therapeutisch vielseitig anwendbar. Somit betrifft die Erfindung ferner Immuntoxine sowie Arzneimittel, die die erfindungsgemäßen Polypeptide bzw. Polypeptiddimere enthalten. Schließlich betrifft die Erfindung diagnostische Zusammensetzungen, die die erfindungsgemäßen Nukleinsäuremoleküle, die erfindungsgemäßen Polypeptide bzw. Polypeptiddimere und/oder Primer, die spezifisch an die erfindungsgemäßen Nukleinsäuremoleküle hybridisieren, enthalten.

Mistelextrakte werden seit Jahrhunderten therapeutisch genutzt. Seit Anfang dieses Jahrhunderts werden Mistelpräparate zur Krebstherapie mit unterschiedlichem Erfolg angewandt [Bocci, 1993; Gabius et al., 1993; Gabius & Gabius, 1994; Ganguly & Das, 1994]. Hajto et al. [1989, 1990] konnte zeigen, daß die therapeutischen Effekte insbesondere durch sogenannte Mistellektine (Viscumine, *Viscum album* Agglutinine, VAA) vermittelt werden. Es wird neben einer cytotoxischen Wirkung heute insbesondere eine (unspezifische) Immunstimulation diskutiert, deren positive Effekte zur begleitenden Therapie und zur Nachsorge von Tumorpatienten ausgenutzt werden. Eine Steigerung der Lebensqualität bei solchen Patienten wird möglicherweise durch die Ausschüttung körpereigener Endorphine vermittelt [Heiny und Beuth, 1994].

Zahlreiche Untersuchungen *in vitro* [Hajto et al., 1990; Männel et al., 1991; Beuth et al., 1993] und *in* vivo [Hajto, 1986; Hajto et al., 1989, Beuth et al., 1991; Beuth et al., 1992], sowie klinische Studien [Beuth et al., 1992] belegen die erhöhte Freisetzung von inflammmatorischen Cytokinen (TNF-a, IL-1, IL-6) sowie eine Aktivierung von zellulären Komponenten des Immunsystems (T_{H}-Zellen, NK-Zellen).

Als aktives Prinzip der Mistelextrakte wird heute ein 60kDa Mistellektin-Protein angesehen, das auf biochemischen Weg aus Extrakten gewonnen werden kann [Franz et al., 1977; Gabius et al., 1992]. Das ML-Protein besteht aus zwei kovalent S-S verbrückten Untereinheiten, dessen A-Kette für eine enzymatische Inaktivierung von Ribosomen [Endo et al., 1988] und dessen B-Kette für die Carbohydratbindung verantwortlich ist. Die biologische Aktivität wird hauptsächlich auf die Lektinaktivität der B-Kette zurückgeführt [Hajto et al., 1990].

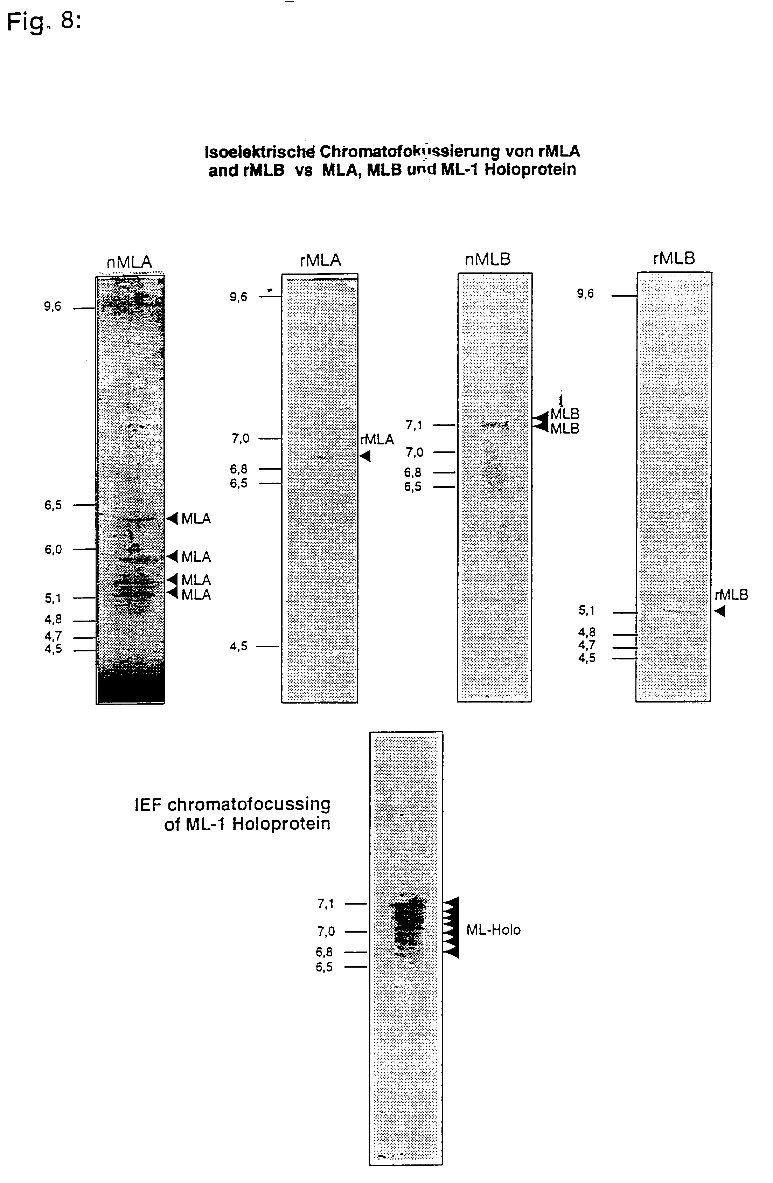

Das Wissen um Struktur-Wirkungsbeziehungen des Mistellektins (ML) ist jedoch bis heute gering. So ist der Beitrag der einzelnen Ketten und deren unterschiedliche biochemische und enzymatische Aktivitäten zur beobachteten Wirkung bzw. den therapeutischen Effekten unklar. Erschwert wird die Analyse der Struktur-Wirkungsbeziehungen durch Kontamination der Präparationen mit anderen Pflanzeninhaltsstoffen der Mistel [Stein & Berg, 1994]. Es wird eine Abhängigkeit der Aktivität von Extraktpräparaten von unterschiedlichen Zusammensetzungen der Extrakte, wiederum abhänging von der Art des Wirtsbaums (z.B. Apfel, Kiefer, Pappel) diskutiert [Hülsen et al., 1986]. Sowohl für Viscotoxine [Garcia-Olmedo et al., 1983; Mendez et al., 1990] als auch für die weiteren Viscumine (z. B. ML-2, ML-3) werden ähnliche Wirkungen postuliert [Eifler et al., 1994]. Selbst auf biochemischen Weg (Affinitätschromatographie) hochrein aufgereinigte ML Präparationen weisen eine bedeutende Heterogenität (Fig. 8) auf. Diese Heterogenität bezieht sich auf die biochemisch meßbaren Aktivitäten der Ketten, auf die hervorgerufenen *in vitro* und *in vivo* Effekte, wie auch auf die Proteinstrukturen selbst. Strukturvarianten werden für Glycosylierungen der ML A- und B-Kette sowie für Sequenzvariationen diskutiert. Gabius et al. [1992] und Dietrich et al. [1992] zeigen eine Sequenzvariabilität der A1 und A2-Ketten des ML-1.

Um die therapeutischen Wirkungen des Mistellektins genauer untersuchen zu können, ist dessen Reindarstellung als strukturell homogene Substanz wünschenswert. Ferner ist für die Fachwelt von Interesse, Mistellektin oder seine Bestandteile in großen Mengen in reiner Form herstellen zu können, um es/sie beispielsweise großtechnisch als wirksamen Bestandteil von Arzneimitteln einsetzen zu können. Diese Ziele konnten bisher durch die im Stand der Technik bekannten Verfahren nicht annähernd realisiert werden. Bei Isolierungen aus pflanzlichem Material wird nach dem derzeitigen Stand der Technik immer ein heterogenes Substanzgemisch erhalten.

Aufgabe der vorliegenden Erfindung war somit, Mistellektin in reiner Form und in Mengen zur Verfügung zu stellen, die eine großtechnische Verwertung erlauben. Die Aufgabe wird durch die in den Ansprüchen gekennzeichneten Ausführungsformen gelöst.

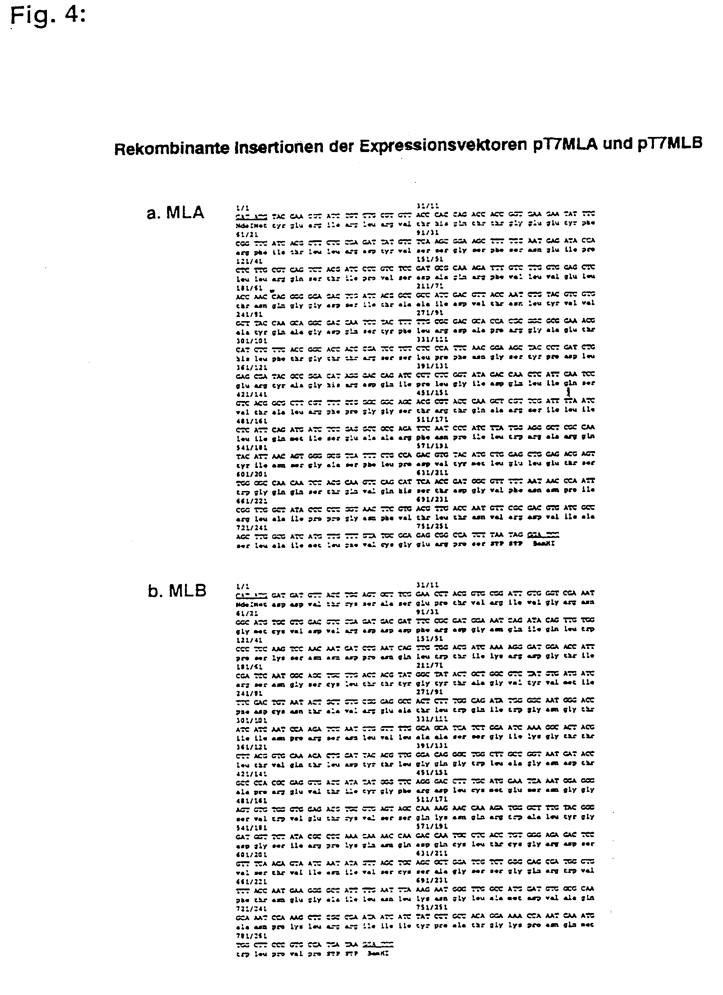

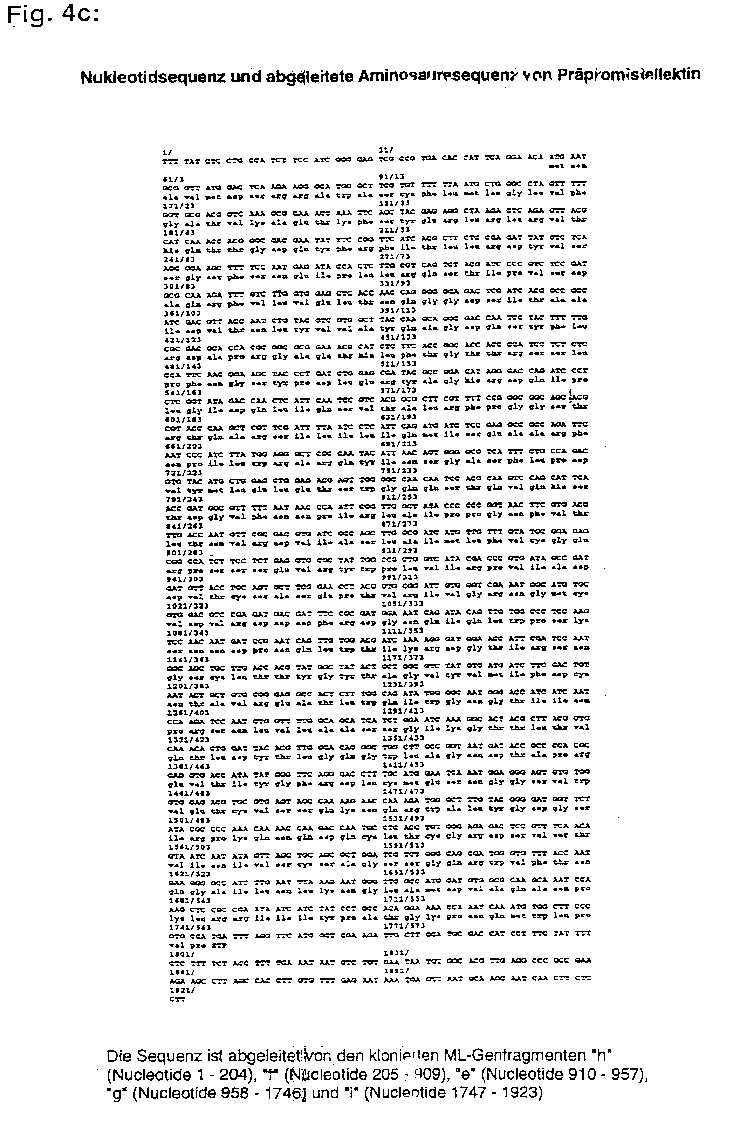

Somit betrifft die Erfindung ein Nukleinsäuremolekül, das (a) ein Präproprotein codiert, das nach Reifung die biologische Funktion des Mistellektindimers aufweist und die in Fig. 4c dargestellte Nukleotidsequenz aufweist; (b) ein Fragment des Präproproteins gemäß (a) codiert, wobei das Fragment natürlicherweise ein biologisch aktiver Bestandteil des Mistellektindimers ist; (c) sich durch die Degeneration des genetischen Codes vom Nukleinsäuremolekül gemäß (a) oder (b) unterscheidet; oder (d) unter stringenten Bedingungen mit dem Nukleinsäuremolekül gemäß (a), (b) oder (c) hybridisiert und ein Polypeptid mit der in (a) oder (b) angegebenen biologischen Funktion codiert.

In der Darstellung von Gensequenzen des Mistellektins können erstmals ausgehend von dieser Sequenz rekombinante, hochreine Einzelketten (rMLA, rMLB) erhalten werden, die *in vitro* reassoziert werden können und so ein rML-Holoprotein ergeben, das proteinchemisch, enzymatisch und strukturell homogen ist. Das reassoziierte rekombinante Protein weist keine Variabilität und Mikroheterogenität, insbesondere hinsichtlich der Primärstruktur und der posttranslationalen Modifikationen (Glycosylierungen, Phosphorylierungen) auf und ist sowohl als Holoprotein, als Teilkette und in Form von Subfragmenten für therapeutische Zwecke besonders geeignet.

Unter einem "Fragment" eines Mistellektinpräproteins wird erfindungsgemäß ein natürlicherweise vorkommendes Fragment verstanden, das ein biologisch aktiver Bestandteil des Mistellektindimers ist.

Der Begriff "natürlicherweise" in Verbindung mit "Bestandteil des Mistellektins" bedeutet erfindungsgemäß, daß das so gekennzeichnete Fragment entweder eine Kette des Mistellektindimers darstellt oder ein Subfragment der Kette ist, das natürlicherweise in der Kette vorkommt. Diese Fragmente sind vorzugsweise biologisch aktiv.

Unter "biologisch aktiv" wird erfindungsgemäß verstanden, daß diese Fragmente mindestens eine biologische Funktion der Ketten oder des Dimers wie in dieser Anmeldung beschrieben oder eine sonstige biologische Funktion der einzelnen Ketten oder des Dimers aufweisen. Außerdem wird unter "biologisch aktiv" auch eine pharmakologische Aktivität verstanden.

Mit Hilfe rekombinanter ML-Proteine ist zusätzlich die Untersuchung der Beiträge der einzelnen Domänen und Subdomänen experimentell erstmals möglich. Rekombinante ML-Proteine und rekombinante Untereinheiten/Teilketten sind die Grundlage entsprechender definierter Monosubstanzpräparate als Ersatz von Extraktpräparaten und standardisierten Extrakten.

Die Klonierung des Mistellektin kodierenden Gens konnte auf der Basis einer neuen Klonierungsstrategie überraschenderweise bewerkstelligt werden, nachdem konventionelle Klonierungsstrategien versagt hatten:

Vom Mistellektin ML-1 sind eine Reihe von proteinchemischen Daten bekannt. Es sind dies neben Molekulargewicht und Untereinheitenstruktur insbesondere kurze N-terminale Peptide, deren Aminosäuresequenzen unabhängig von Dietrich et al. [1992] und Gabius et al. [1992] [siehe auch DE4221836] beschrieben worden sind. Es ist unmöglich, ausgehend von den N-terminalen Peptiden der A- bzw. B-Kette aufgrund von deren Aminosäurezusammensetzungen und dem damit verbundenen hohen Degenerationsgrad der ableitbaren Nukleinsäuresequenzen synthetische Oligonukleotide herzustellen, deren Degenerationsgrad ausreichend niedrig ist, um beim Screening von genomischen Genbibliotheken zur Identifikation von ML-Genfragmenten zu kommen. Dies gilt ebenfalls für cDNA-Genbanken, die ausgehend von *Viscum album* poly-(A+) RNA dargestellt wurden.

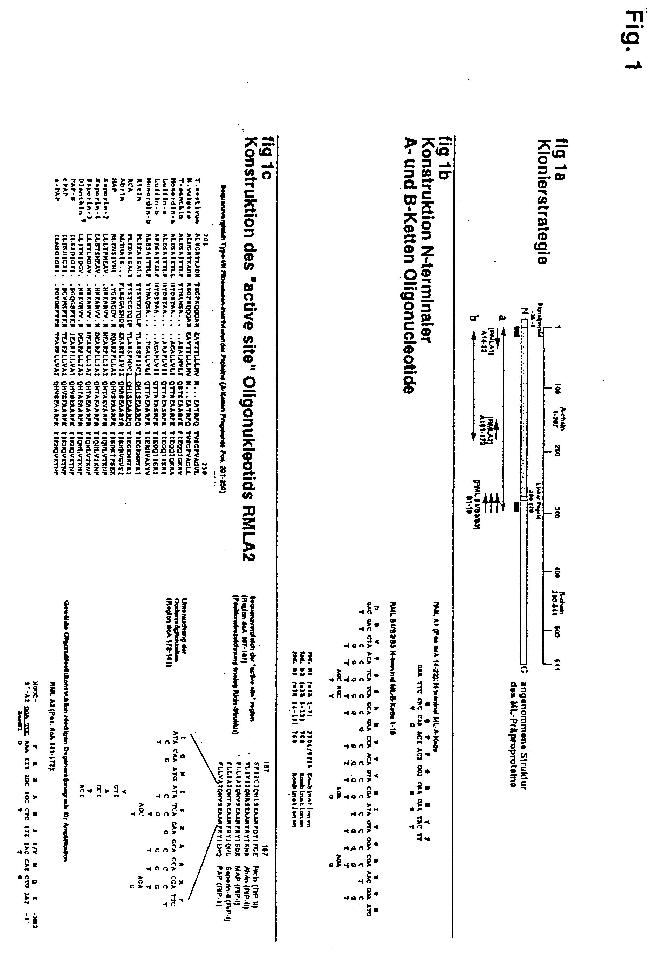

Die Polymerase-Kettenreaktion erlaubt die Amplifikation von DNA-Abschnitten, die zwischen bekannten Abschnitten liegen [Erlich et al., 1988]. Mit einem "sense"-Oligonukleotid ausgehend von N-Terminus von MLA und einem "antisense" Oligonukleotid N-terminal von MLB wäre eine Amplifikation des dazwischenliegenden Genabschnitts unter der Vorraussetzung der Intronfreiheit des ML-Gens denkbar (Fig. 1a). In der Praxis zeigt eine Analyse der N-terminalen Sequenz der B-Kette, daß der Degenerationsgrad der denkbaren Kombinationen von Oligonukleotiden für eine erfolgreiche Durchführung dieses Ansatzes jedoch viel zu hoch ist. Dies begründet sich insbesondere aus der für eine Oligonukleotidkonstruktion ungünstigen Sequenz des B-Ketten N-Terminus, wodurch eine Amplifikation von ML-Gensequenzen ausgehend von den bekannten Aminosäuresequenzbereichen nicht praktikabel ist (Fig. 1b).

Zur Klonierung des ML-Gens mit Hilfe einer veränderten PCR-Strategie wurde daher versucht, zur Konstruktion von Amplifikationsoligonukleotiden weitere Proteindaten, insbesondere auf der Basis von Verwandschaftsbeziehungen des Mistellektins zur Klasse der Typ I und Typ II Ribosomen-inaktivierender Proteine (RIPs) [Stirpe et al., 1992] einfließen zu lassen. Basierend auf multiplen Alignments von (a) Typ I RIP-Proteinen und Ricin-A-Ketten sowie (b) den B-Ketten von Abrin und Ricin wurden an insgesamt 8 Sequenzbereichen konservierte Regionen identifiziert. Ausgehend von diesen Sequenzregionen und unter Einbeziehung von Codonnutzungstafeln verwandter Spezies wurden insgesamt 21 Oligonukleotide konstruiert und in verschiedenen Kombinationen zu mehr als 200 Amplifikationsexperimenten eingesetzt. In keinem Fall konnten jedoch spezifische Amplifikationsprodukte erhalten werden, obwohl die PCR-Bedingungen, was Annealing-Temperatur, Mg²⁺-Gehalt sowie Zyklusparameter betrifft, in weiten Grenzen variiert wurde.

Sowohl Screening von genomischen und cDNA-Banken als auch die Anwendung von PCR-Techniken erlaubten es somit nicht, aufgrund der angeführten Überlegungen zu spezifischen ML-DNA-Sequenzen zu gelangen.

Es wurde daher nach neuen Wegen gesucht, weitere strukturelle Eigenschaften der Ricin- und Abrin- Struktur in die Konstruktion der Amplifikations-oligonukleotide einfließen zu lassen.

Da der enzymatische Mechanismus von Ribosomen inaktivierenden Proteinen (RIPs), hier insbesondere dem TypII-RIP Ricin, dem des ML gleicht [Endo et al., 1988a +1988b], war nicht auszuschließen, daß auch strukturelle Übereinstimmungen auf der Ebene der funktionellen Primär- und Tertiärstrukturbereiche vorhanden sind. Ausgehend von der Kristallstruktur von Ricin [Katzin et al., 1991; Rutenber & Robertus, 1991; Weston et al., 1994] ergab eine Analyse der Kettenflexibilitäten in der Ricin-A Kette einen Hinweis auf die geringe Mobilität des Arg180, das innerhalb eines konservierten Sequenzbereiches liegt. Weiterhin wurde eine Analyse der aufgrund der sterischen Anordnung des Kettenrückgrats gegebenen möglichen Aminosäuresubstitutionen in diesem Bereich des aktiven Zentrums unter Berücksichtigung der Substrat-wechselwirkungen durchgeführt. Die Ergebnisse dieser Überlegungen wurden nun mit einer Auswertung von umfassenden Sequenzalignments der Ricin-A-Kette und weiteren TypI RIPs in Bezug gebracht.

Mit der Erweiterung der Ergebnisse der Sequenzvergleiche durch die Einbeziehung struktureller Daten ergaben sich so Wahrscheinlichkeitsdaten für das Auftreten bestimmter Aminosäurereste an bestimmten Positionen. Dadurch wurde es möglich, eine Reihe von theoretischen ML-Aminosäuresequenzen für diesen Bereich zu postulieren und anhand dieser ein entsprechendes außergewöhnlich gering degeneriertes Oligonukleotide (RMLA2) zu konstruieren (Fig. 1c).

Es konnten nun mit der Kombination RMLA1 (ein degeneriertes Oligonukleotid, das aus der N-terminalen Aminosäuresequenz der MLA-Kette abgeleitet wurde; vgl. Fig. 1c) und dem durch obige Überlegungen konstruierten "active-site" Oligonukleotid RMLA2 bei definierten PCR-Parametern ausgehend von komplexer genomischer ML-DNA Fragmente erhalten werden, nachdem alle diesbezüglichen alternativen Ansätze wie oben beschrieben ohne Ergebnis blieben.

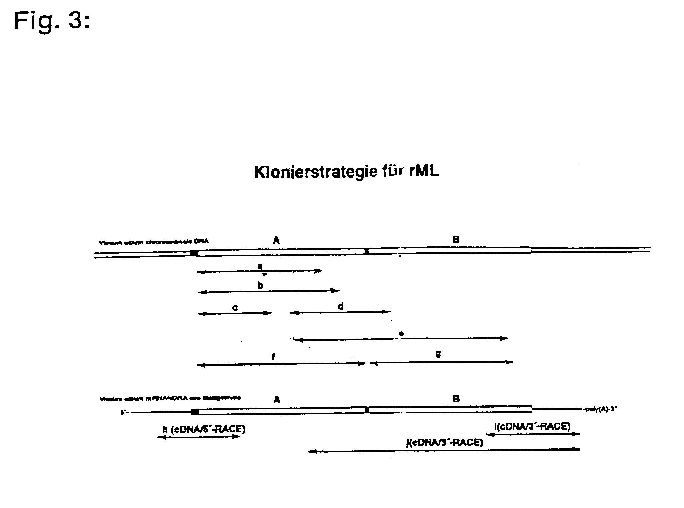

Die Vervollständigung der Sequenzinformation des Gens wurde nun über spezifische nichtdegenerierte Oligonukleotidprimer, abgeleitet aus der mit Klonierung und Sequenzierung des Fragments a (Fig. 3) vorhandenen Genteilsequenz des MLA, und degenerierten Oligonukleotiden, abgeleitet aus RIP I und Ricin/Abrin Sequenzalignments, mit Hilfe von weiteren PCR-Amplifikationen durchgeführt. Zur Konstruktion der degenerierten B-Ketten-Oligonukleotide wurden Sequenzalignments der B-Ketten der Ricine und Abrine herangezogen, wo ebenfalls einige Bereiche hoher Konservierung gefunden wurden.

Zur Darstellung der 5'- und 3'-Enden des Holoproteins, B-Ketten-Teilfragmente sowie der 5'- und 3'- nichttranslatierten Abschnitte wurde ausgehend von isolierter Mistel-RNA analoge cDNA durch reverse Transkription synthetisiert und mit Hilfe der RACE-Technik [Frohman et al., 1988] die entsprechenden Genabschnitte dargestellt. Nachdem eine Vielzahl sich jeweils überlappender Genfragmente vorhanden war (Fig. 3), wurden anschließend vollständige A-Ketten- und B-Ketten-Genabschnitte, jeweils ausgehend von komplexer genomischer Mistel-DNA wiederum durch spezifische PCR dargestellt. Die Gensequenzen von rMLA und rMLB, versehen mit terminalen Modifikationen, sind in Fig. 4a und Fig. 4b dargestellt. Die auch 5'- und 3'- nichttranslatierte Bereiche sowie Endopeptid- und Signalpeptid-codierende Genabschnitte umfassende vollständige ML-Gensequenz ist in Fig. 4c dargestellt.

In einer bevorzugten Ausführungsform des erfindungsgemäßen Nukleinsäuremoleküls ist das Fragment die A-Kette des Mistellektins, die durch die in Fig. 4a (MLA) dargestellte Nukleotidsequenz kodiert wird.

In einer weiteren bevorzugten Ausführungsform des erfindungsgemäßen Nukleinsäuremoleküls ist das Fragment die B-Kette des Mistellektins, die durch die in Fig. 4b (MLB) dargestellte Nukleotidsequenz kodiert wird.

Eine weitere bevorzugte Ausführungsform der Erfindung betrifft ein Nukleinsäuremolekül, das ein DNA-Molekül ist.

Erfindungsgemäß wird unter "DNA-Molekül" sowohl ein genomisches, wie auch ein cDNA-Molekül oder ein (semi)synthetisches DNA-Molekül verstanden. Verfahren zur Herstellung dieser verschiedenen DNA-Moleküle sind dem Fachmann in Kenntnis der erfindungsgemäßen Lehre bekannt.

In einer weiteren bevorzugten Ausführungsform der Erfindung ist das Nukleinsäuremolekül ein RNA-Molekül.

Die Erfindung betrifft ferner einen Vektor, der mindestens ein erfindungsgemäßes Nukleinsäuremolekül enthält.

Der erfindungsgemäße Vektor kann beispielsweise ein einziges erfindungsgemäßes Nukleinsäuremolekül enthalten, das das gesamte Mistellektin-Präproprotein kodiert. Sofern dieser Vektor ein Expressionsvektor ist, kann das Präproprotein in einem geeigneten transformierten Wirt prozessiert werden und die monomeren Einheiten zum Mistellektindimer *in vivo* oder *in vitro* zusammengefügt werden. In einer anderen Ausführungsform ist der erfindungsgemäße Vektor ein Vektor, der lediglich zur Propagation der erfindungsgemäßen Nukleinsäure eingesetzt wird.

In einer bevorzugten Ausführungsform enthält der erfindungsgemäße Vektor sowohl ein Nukleinsäuremolekül, das die A-Kette des Mistellektins oder ein Fragment davon kodiert, als auch ein Nukleinsäuremolekül, das die B-Kette oder ein Fragment davon kodiert, wobei die Fragmente der Monomere biologisch aktiv sind.

In einer weiteren bevorzugten Ausführungsform ist der erfindungsgemäße Vektor ein Expressionsvektor. Dem Fachmann ist klar, wie er geeignete Expressionsvektoren für verschiedene Wirtsorganismen bereitstellt.

Erfindungsgemäß wurde zur heterologen Expression eine für die Mistellektin A-Kette codierende Sequenz durch spezifische PCR ausgehend von komplexer genomischer Mistel-DNA dargestellt. Über nicht-komplementäre Bereiche der eingesetzten Primer-Oligonukleotide wurden hierbei Translations-Kontrollelemente sowie Erkennungssequenzen von Restriktionsendonukleasen angefügt, wodurch ausgehend von dem genomisch vorliegenden Präpromistellektin-Gen die Klonierung und getrennte Expression der Mistellektin A-Kette ermöglicht wurde.

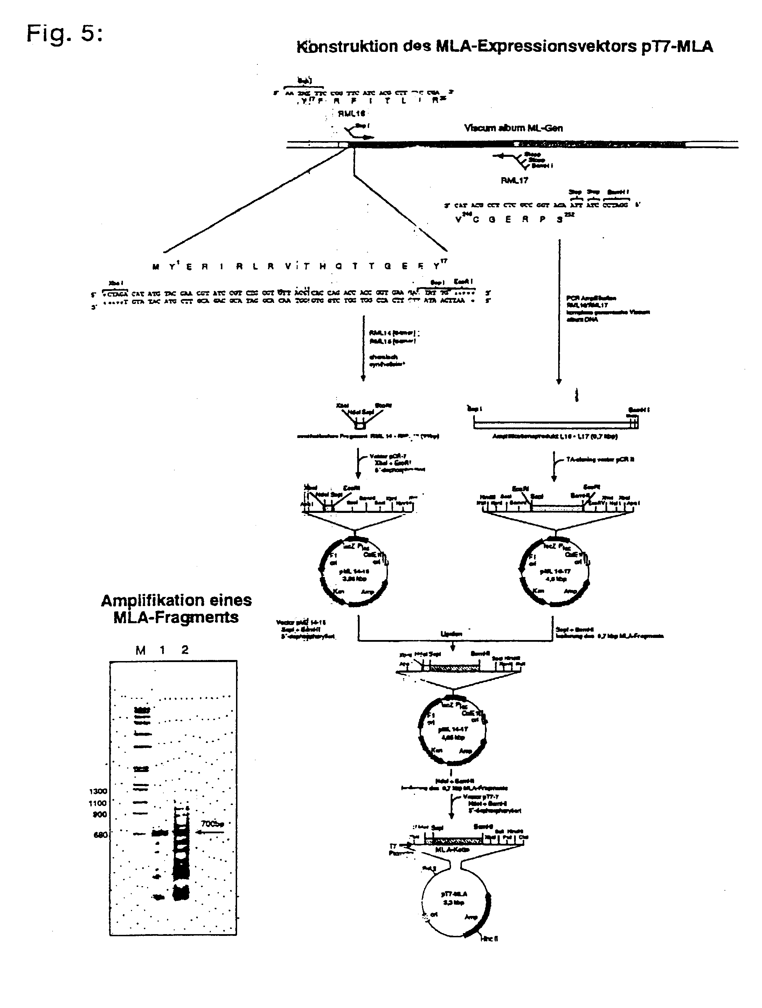

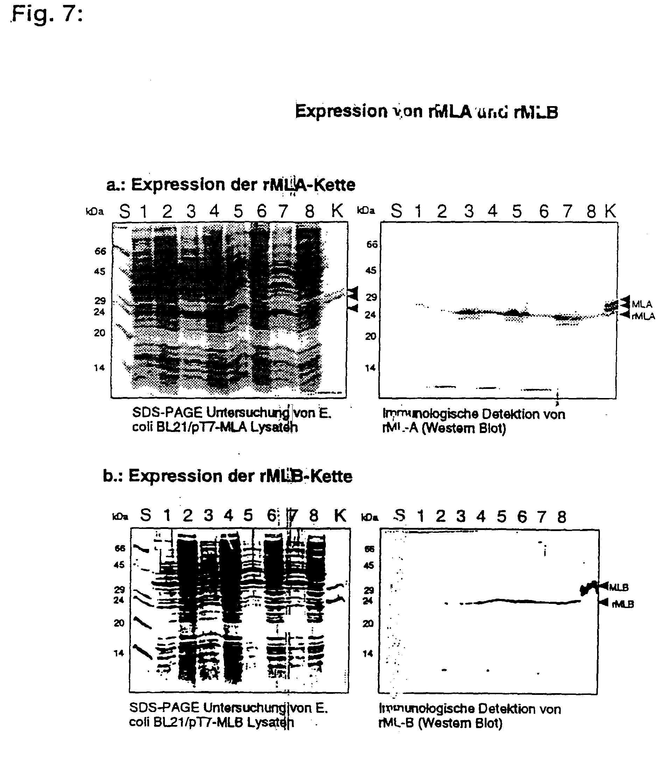

Der 5'-Bereich der für rMLA codierenden Sequenz entsprechend der Aminosäurereste Tyrosin¹ - Tyrosin¹⁷ [Dietrich et al., 1992; Gabius et al., 1992] wurde unter Vorschaltung eines Translations-Startcodons als synthetisches Genfragment durch Hybridisierung und Klonierung zweier Oligonukleotide dargestellt. Hierdurch wurde eine Optimierung der Gensequenz hinsichtlich der Codon-Auswahl, wie sie für stark exprimierte Gene in *Escherichia coli* beschrieben ist [Gribskov et al., 1984], erzielt. Am 3'-Ende des synthetischen rMLA-Genfragments sowie am 5'-Ende des mittels PCR dargestellten rMLA-Genfragments wurde durch den gezielten Austausch des Tyrosin¹⁷-Codons von TAC zu TAT eine *Ssp* I - Restriktionsschnittstelle eingeführt, die eine Fusion der beiden rMLA-Genfragmente unter Darstellung des Vektors pML14-17 (Fig. 5) ermöglichte. Die für rMLA codierende Sequenz wurde durch DNA-Sequenzierung bestätigt (Fig. 4a). Zur Expression von rMLA in *Escherichia coli* wurde die Gensequenz aus dem Vektor pML14-17 isoliert und durch Insertion in den Expressionsvektor pT7-7 [Studier & Moffart, 1986] unter die Kontrolle des T7-RNA Polymerase Promotors sowie eines Transkriptionsterminators gestellt. Mit dem resultierenden Expressionsvektor pT7-MLA (Fig. 5) wurde der *E. coli* Expressionsstamm BL21 transformiert. Die Induktion der Genexpression ist durch das Auftreten einer Proteinbande entsprechend der nicht-glykosylierten, rekombinanten Mistellektin A-Kette gekennzeichnet, die eine relative Molekülmasse von 25 kDa besitzt. Nachweis und Identifizierung des rekombinanten Expressionsproduktes erfolgte durch Western-Blot Analyse unter Verwendung eines spezifischen anti-MLA-Antikörpers (Fig. 7).

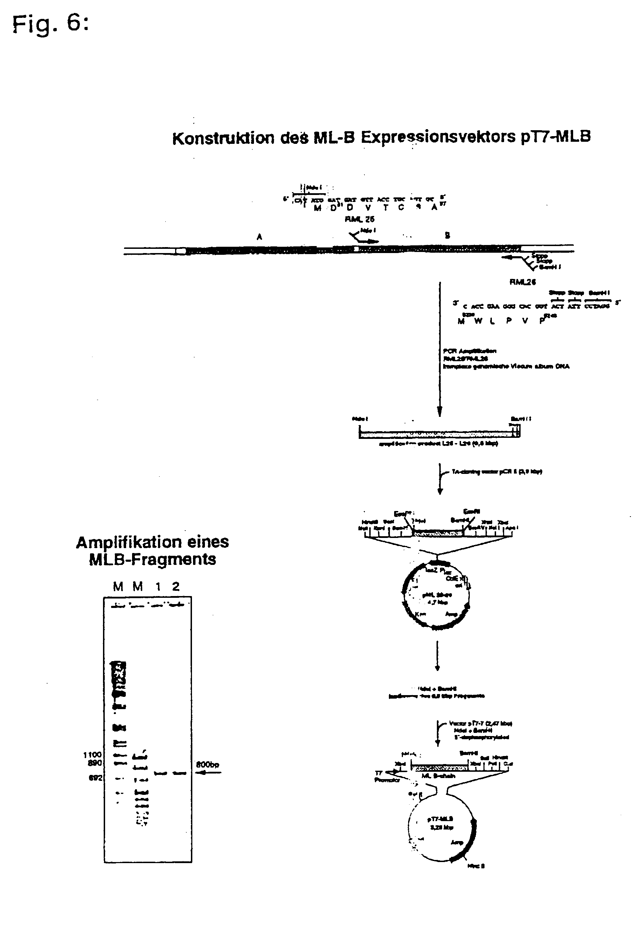

Zur heterologen Expression der Mistellektin B-Kette wurde die vollständige, MLB codierende Sequenz durch spezifische PCR aus komplexer genomischer *Viscum album* DNA amplifiziert, wobei über nicht-komplementäre Bereiche der eingesetzten Primer-Oligonukleotide Translationskontrollelemente sowie Erkennungssequenzen für Restriktionsendonukleasen eingeführt wurden (Fig. 6). Das resultierende 0,8 kbp PCR-Produkt wurde nach Klonierung im TA-Klonierungsvektor pCRII durch Insertion in den Expressionsvektor pT7-7 unter die Kontrolle von Transkriptionskontrollelementen gestellt und mit dem resultierenden Expressionsvektor pT7-MLB der Expressionsstamm *E. coli* BL21 transformiert.

Die Integrität der rMLB-codierenden Sequenz wurde durch DNA-Sequenzierung bestätigt (Fig. 4b). Der Nachweis der Expression erfolgte durch Western-Blot Analyse unter Verwendung eines spezifischen anti-MLB-Antikörpers (TB33), wobei 2h nach Induktion der Genexpression ein immunreaktives Protein mit einer relativen Molekülmasse von 31 kDa entsprechend der nicht-glykosylierten, rekombinanten Mistellektin B-Kette auftritt (Fig. 7b). Eine Analyse der Zellfraktionen nach Gesamtzellaufschluß der *E. coli* Zellen zeigte eine Aufteilung der synthetisierten rMLB-Kette in einen löslichen Anteil im Überstand sowie einen unlöslichen "inclusion bodies"-Anteil im Sediment des E. *coli* Zellaufschlußes, wobei 4 h nach Induktion der lösliche bzw. unlösliche Anteil jeweils 50 % der Gesamtausbeute betrug (Fig. 7b).

Ferner betrifft die Erfindung einen Wirt, der mit mindestens einem erfindungsgemäßen Vektor transformiert ist wobei der transformierte Wirt kein Mensch, keine transgene Tierart und keine transgene Pflanze und keine transgene Pflanzensorte ist.

Je nach Zielsetzung des Fachmannes kann mit dem erfindungsgemäßen Wirt lediglich eines der beiden Monomere oder die Kombination beider Monomere, vorzugsweise als assoziiertes Dimer hergestellt werden. Der erfindungsgemäße Wirt kann eine eukaryontische oder prokaryontische Zelle oder ein transgenes Tier sein, nicht aber eine transgene Tierart.

Vorzugsweise ist der erfindungsgemäße Wirt eine Säugerzelle, eine Pflanzenzelle, ein Bakterium, eine Pilzzelle, eine Hefezelle oder eine Insektenzelle.

In einer besonders bevorzugten Ausführungsform ist der erfindungsgemäße Wirt das Bakterium E.coli, eine Aspergillus-Zelle oder eine Spodoptera-Zelle, vorzugsweise eine Spodoptera frugiperda-Zelle.

Die Erfindung betrifft ferner ein Polypeptid, das von dem erfindungsgemäßen Nukleinsäuremolekül und vom erfindungsgemäßen Wirt produziert wird, wobei der Wirt keine transformierte Pflanzenzelle ist oder dem erfindungsgemäßen Vektor kodiert wird und vom erfindungsgemäßen Wirt produziert wird, wobei der Wirt keine transformierte Pflanzenzelle ist oder vom erfindungsgemäßen Wirt produziert wird, wobei der Wirt keine transformierte Pflanzenzelle ist.

Das erfindungsgemäße Polypeptid weist die biologische Aktivität der A-Kette oder der B-Kette des Mistellektins auf. In anderen Ausführungsformen kann das erfindungsgemäße Polypeptid jedoch auch nur einen Teil der biologischen Aktivität aufweisen. Unter "Teil der biologischen Aktivität" wird erfindungsgemäß entweder eine verminderte Aktivität und/oder eine Anzahl von Aktivitäten aus dem biologischen Aktivitätsspektrum verstanden. Das erfindungsgemäße Polypeptid kann auch ein Fragment der A- oder B-Kette sein, das eine der vorgenannten Eigenschaften aufweist.

### Untersuchung der Eigenschaften von rMLA und rMLB

### (I) Relative Molekülmassen und Struktur

Die relativen Molekülmassen wurden durch SDS-Polyacrylamidgel-Elektrophorese unter reduzierenden Bedingungen und der anschließenden Proteinfärbung mittels Silber bzw. Coomassie-Brilliant-Blue bzw. durch immunologische Färbung im Rahmen einer Western-Blot Analyse bestimmt.

Überraschenderweise wurde hierbei gefunden, daß die rekombinante, nicht-glykosylierte Mistellektin A-Kette eine relative Molekülmasse von 25 kDa zeigt und sich damit signifikant von den natürlichen Mistellektin A-Ketten A₁ mit 31 kDa bzw. A₂ mit 29 kDa unterscheidet. Dieser Unterschied im relativen Molekulargewicht ist vor allem deshalb überraschend, weil im Stand der Technik davon ausgegangen wurde, daß die A-Kette nicht glykolisiert ist. Die rekombinante Mistellektin B-Kette weist eine relative Molekülmasse von 31 kDa auf und ist damit wesentlich leichter als die glykosylierte, natürliche Mistellektin B-Kette mit 36 kDa. (Fig. 7).

Die bei natürlichen ML-Proteinen auftretende Heterogenität aufgrund Glykosylierung und/oder Sequenzvariationen, die sich im SDS-Gel als breite Bande zeigt, tritt im Falle der rekombinanten Spezies in keinem Fall auf.

Die relativen Molekülmassen des reassoziierten rMLA /rMLB Holoproteins addieren sich zu 56 kDa im Vergleich zu dem schwereren natürlichen ML (nML) mit 65 - 67 kDa.

### (II) Isoelektrische Homogenität

rMLA erweist sich als isoelektrisch homogenes Protein mit einen isoelektrischen Punkt von 6,8 im Gegensatz zu hochreinen natürlichen Mistellektin A-Ketten, die sich auf 4 Species mit isoelektrischen Punkten von 5,2; 5,4; 5,7 und 6,2 aufteilen. (Fig. 8).

rMLB erweist sich als isoelektrisch homogenes Protein mit einen isoelektrischen Punkt von 5,1 im Gegensatz zu natürlicher Mistellektin B-Kette, die sich auf mindestens 2 Species mit isoelektrischen Punkten von 7,1 und 7,3 aufteilt. (Fig. 8).

Es ergeben sich damit für das natürliche ML-Holoprotein eine Vielzahl von Molekülvarianten und -kombinationen (Fig. 8 unten), während sich für rMLA,B Proteine eine einheitliche Mobilität in der IEF Chromatofocussierung ergibt, was die Homogenität des rML gegenüber der Mikroheterogenität der natürlichen Proteinspezies dokumentiert.

### (III) Enzymatische Aktivität von rMLA

Durch Einsatz immunaffinitätsgereinigter rMLA Präparationen in einem gekoppelten Transkriptions-/Translationsassay wurde translationsinhibierende Aktivität für rMLA (isoliert aus dem löslichem Expressionsproduktanteil) und rMLA (isoliert aus unlöslichem "inclusion-bodies"-Anteil) nachgewiesen.

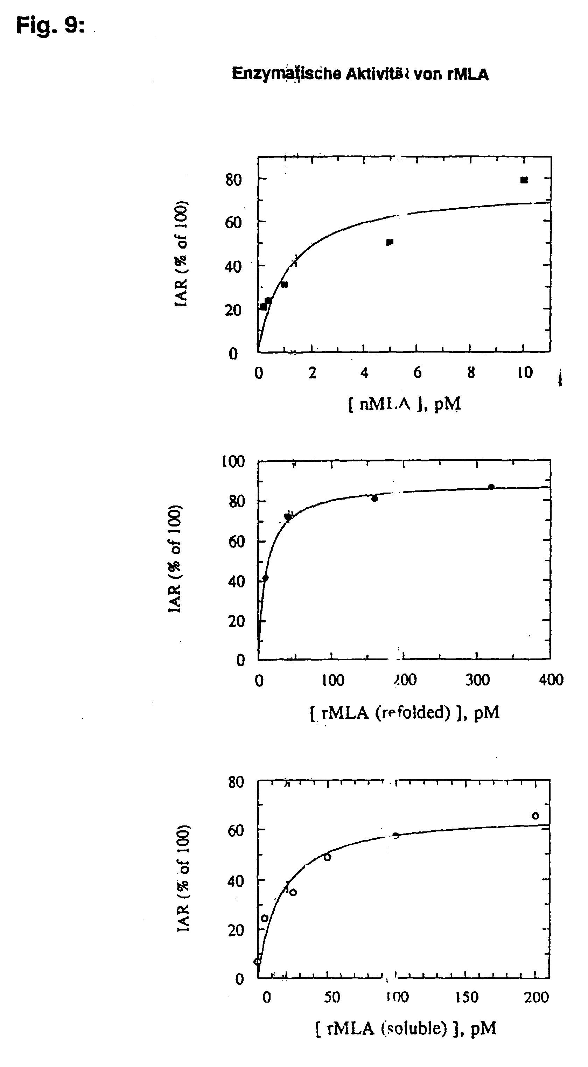

rMLA zeigt im Vergleich zu natürlicher Mistellektin A-Kette eine unterschiedliche Hemmcharakteristik in Bezug auf die Dosisabhängigkeit der Translationsinhibition sowie in Bezug auf die nicht-inhibierbare Translations-Restaktivität im verwendeten Retikulozyten-Lysat. (Fig. 9). Die enzymatische Eigenschaft, die die Grundlage für die toxische Wirkung ML-Holoproteinen darstellt, ist in rekombinanten Spezies signifikant reduziert.

### (IV) Carbohydrat-bindende Aktivität von rMLB

rMLB-Ketten, sowohl aus der löslichen wie auch aus der renaturierten/reoxidierten löslichen Expressionsfraktion sowie reassoziierte rMLA/rMLB, rMLA/MLB und MLA/rMLB Holoproteine können an eine Lactosylsepharosesäule (Säulenpuffer 20 mM Phosphat-Puffer, 50 mM NaCl, 1 mM EDTA, pH 7,2) gebunden und spezifisch mit Lactose (0,3 M Lactose in Säulenpuffer) eluiert werden. Die Carbohydratbindeaktivität der rekombinanten Proteine kann so zur Affinitätsreinigung herangezogen werden.

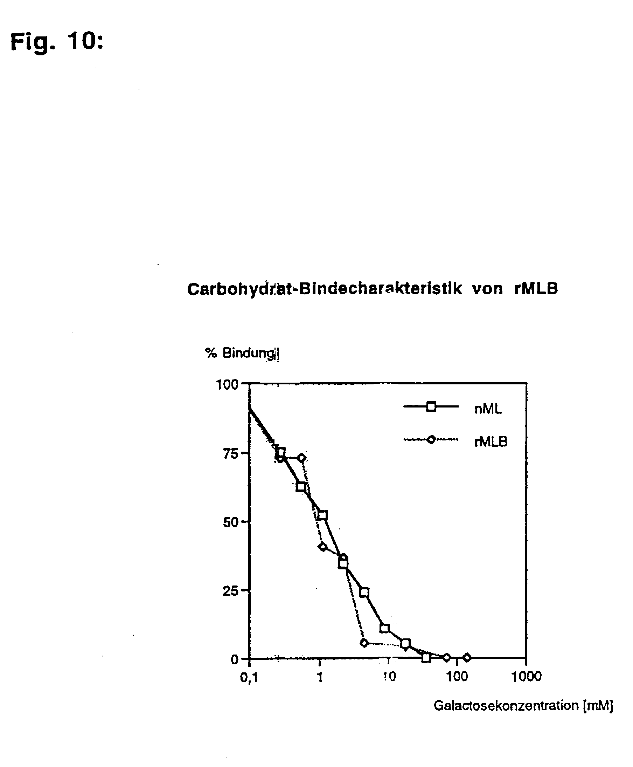

Eine Quantifizierung der Carbohydratbindung durch rekombinante rMLB-Proteine kann durch Messung im ELLA-System unter kompetitiven Bedingungen mit Galactose oder Lactose erfolgen. Der kompetitive ELLA-Test zeigt Unterschiede in der Bindecharakteristik, ausgedrückt durch die systemspezifischen IC₅₀-Werte für die halbmaximale Verdrängung der Proteine vom immobilisierten Asialofetuin als Ligand durch Galactose (IC₅₀ nML: 1,65 mM, IC₅₀ rMLB: 1,3 mM) (Fig. 10).

In einer bevorzugten Ausführungsform weist das erfindunsgemäße Polypeptid mindestens eine chemische oder enzymatische Modifizierung auf, wobei die enzymatische Modifizierung keine in Viscum album vorkommende Glykosilierung ist.

Diese Modifizierung kann die gegebenenfalls vorhandene biologische Aktivität des Polypeptids verändern, erniedrigen oder erhöhen. Eine derartige Modifizierung kann z.B. nach der Translation und Isolierung des Polypeptids erfolgen. Andererseits können derartige Modifizierungen bei der chemischen oder semisynthetischen Herstellung des erfindungsgemäßen Polypeptids eingefügt werden. Diese Modifikationen können vom Fachmann nach an sich bekannten Verfahren eingefügt werden, um die pharmakologische Aktivität des Mistellektins zu verändern, vorzugsweise zu verbessern.

In einer weiteren bevorzugten Ausführungsform ist das erfindungsgemäße Polypeptid ein Fusionsprotein. Das Fusionsprotein weist vorzugsweise die vorstehend definierte biologische Aktivität auf.

Auch diese Ausführungsform des erfindungsgemäßen Polypeptids ist vorzugsweise darauf ausgerichtet, die pharmakologischen Eigenschaften der Mistellektin-Polypeptide für weitere Targets auf zellulärer Ebene zu verändern und vorzugsweise zu verbessern.

Die Erfindung betrifft ferner ein Polypeptiddimer mit den biologischen Aktivitäten des Mistellektins, wobei die beiden Monomere von den erfindungsgemäßen Nukleinsäuremolekülen codiert werden und wobei mindestens eines der Monomere vom erfindungsgemäßen Wirt produziert wird, wobei der Wirt keine transformierte Pflanzenzelle ist.

Unter "biologische Aktivität des Mistellektins" wird jede biologische Aktivität aus dem Spektrum der gesamten biologischen Aktivitäten des Mistellektins verstanden. Eine derartige Funktion ist z.B. die pharmakologische Wirkung des Mistellektins.

In zahlreichen humanen und murinen Tumorzellinien induzierte pflanzliches Mistellektin-1 den Zelltod durch apoptotische Mechanismen (Janssen, 1993). Mistellektin-1 bzw. die B-Kette alleine induzierten die Freisetzung von Cytokinen aus peripheren mononukleären Zellen gesunder, humaner Blutspender (Hajto, 1990). Aus neutrophilen Granulocyten von Krebspatienten induzierte Mistellektin-1 die Sekretion von Superoxidanionen (Timoshenko, 1993). Mistellektin-1 induzierte die Expression der a-Kette des Interleukin-2-Rezeptors (CD25) bzw. des HLA-DQ-Antigens auf peripheren Lymphozyten gesunder, humaner Blutspender (Beuth, 1992). Nach Applikation von Mistellektin-1 in Mäuse wurde eine Zunahme der Thymuszellzahl, der Anzahl der zytotoxischen T-Lymphozyten (Lyt-2+) und der Helfer-T-Zellen (L3T4+) im Thymus und die Zahl der Peritonealmakrophagen, auch speziell derjenigen, die den Aktivierungsmarker MAC-3 trugen, gemessen (Beuth, 1994). Auch das Verhältnis L3T4+/Lyt2+ im Thymus der Versuchstiere wurde erhöht. Im peripheren Blut der Mäuse wurde die Dichte der Leukozyten, Lymphozyten, Monozyten im allgemeinen und speziell der Lymphozyten, die den Interleukin-2-Rezeptor als Aktivierungsmarker auf der Zelloberfläche exprimieren, sowie der Monozyten, die den Aktivierungsmarker MAC-3 exprimierten, nach Behandlung mit Mistellektin-1 gesteigert (Beuth, 1994). Im Blut von Krebspatienten erhöhte Mistellektin-1 die Dichte der T-Lymphozyten (CD4+, CD8+), der natürlichen Killerzellen und der B-Lymphozyten (Beuth, 1992). Ferner wurde eine Erhöhung des endogenen Opiatmediators β-Endorphin im Blutplasma von Mammakarzinompatientinnen nach Applikation von Mistellektin-1 nachgewiesen (Heiny, 1994). Zudem wurde nachgewiesen, daß Mistellektin-1 die zytotoxische Wirkung von peripheren, natürlichen Killerzellen gegenüber K-562-Tumorzellen und die Dichte von großen, granulären Lymphozyten (LGL) im peripheren Blut erhöht (Hajto, 1989). Eine antimetastatische Aktivität von Mistellektin-1 auf Sarkom-Zellen in Mäusen wurde belegt (Beuth, 1991).

Vorzugsweise weist das erfindungsgemäße Polypeptiddimer dasselbe Spektrum an biologischen Aktivitäten wie das natürliche Mistellektindimer auf.

Zur Darstellung von rML-Holoprotein aus den getrennt rekombinant synthetisierten Einzelketten wurde rMLB mit einem molaren Überschuß rMLA in Anwesenheit eines Glutathion Redox-Systems und Protein-Disulfid-Isomerase *in vitro* reassoziiert. Das dem Heterodimer entsprechende rML- Holoprotein wurde unter Abtrennung freier rMLA und rMLA-Dimere durch Affinitätschromatographie an Lactosyl-Agarose aus dem Reassoziationsansatz isoliert und gereinigt. Mit analogen Vorgehen sind rMLA/MLB und MLA/rMLB heterodimere Holoproteine darstellbar.

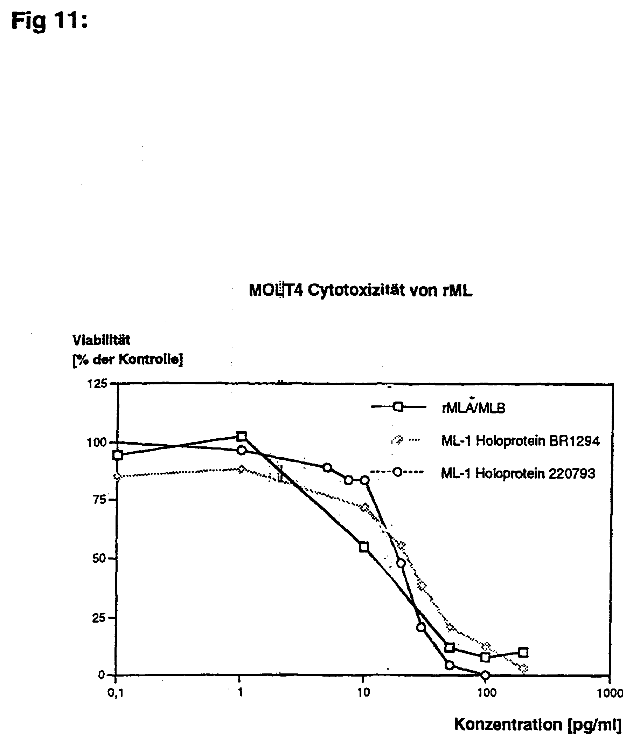

Die Wirkung reassoziierter rMLA/MLB Holoproteine wurde durch Verfolgung der cytotoxischen Aktivität auf eine humane Monocytenleukämiezellinie (MOLT4) getestet. Sowohl B-Kette (Oberflächenbindung) als auch A-Kette (enzymatische Ribosomeninaktivierung) leisten Beiträge zum beobachteten cytotoxischen Effekt. Ein *in vitro* reassoziiertes rMLA/MLB Holoprotein wurde mit zwei Chargen natürlichem ML-Holoprotein verglichen und zeigt vergleichbar hohe cytotoxische Eigenschaften mit IC₅₀-Werten um 10pg/ml (Fig. 11), was die funktionelle Integrität des Reassoziats dokumentiert.

In einer weiteren Ausführungsform weist das erfindungsgemäße Polypeptiddimer mit den biologischen Aktivitäten des Mistellektins, wobei die beiden Monomere von den erfindungsgemäßen Nukleinsäure molekülen codiert werden als mindestens eines der Monomere ein chemisch oder enzymatisch modifiziertes erfindungsgemäßes Polypeptid oder ein erfindungsgemäßes Fusionsprotein auf, wobei bei mindestens einem des Monomere die enzymatische Modifizierung keine in Viscum album vorkommende Glykosilierung ist.

Durch Modifikationen können zum einen die Wirkungsstärken optimiert werden, als auch durch Ausschaltung einzelner Wirkqualitäten (z.B. Kohlenhydratbindungsstellen der B-Kette oder Glycosidaseaktivität der A-Kette) die therapeutischen Einsatzmöglichkeiten erweitert werden, indem eventuelle Nebenwirkungen ausgeschaltet werden. Polypeptide mit veränderten Eigenschaften können auch als Werkzeuge zur Aufklärung der Wirkmechanismen dienen. Für die Therapie wird es wahrscheinlich notwendig werden, die Antigenität und die Immunogenität der Polypeptide zu vermindern und/oder ihre pharmakokinetischen Eigenschaften zu optimieren.

Die Erfindung betrifft weiterhin ein Verfahren zur Herstellung des erfindungsgemäßen Polypeptids oder Polypeptiddimers, wobei man den erfindungsgemäßen Wirt unter geeigneten Bedingungen züchtet und das so erhaltene Polpeptid oder Polypeptiddimer isoliert.

Dem Fachmann sind geeignete Bedingungen zur Züchtung und Isolierung des Wirtes bekannt. So kann beispielsweise das erfindungsgemäße Polypeptid oder Polypeptiddimer mittels eines geeigneten Expressionssystems aus dem Wirt ausgeschleust und im Medium gesammelt werden. Andererseits können die Polypeptide oder Polypeptiddimere in der Zelle verbleiben und aus dieser isoliert werden. Nachfolgend wird eine weitere bevorzugte Ausführungsform des erfindungsgemäßen Verfahrens vorgestellt:

Zur Isolierung von rMLA erfolgte ein Gesamtzellaufschluß der *E. coli* Zellen und eine Trennung löslicher von unlöslichen Zellbestandteilen durch Zentrifugation. Eine Analyse der Zellfraktionen zeigte, daß die rekombinante Mistellektin A-Kette in Abhängigkeit von den Expressionsbedingungen und der Expressionsdauer zu 5 - 50 % in löslicher Form bzw. zu 50 - 95 % in Form unlöslicher Proteinaggregate ("inclusion bodies") akkumuliert.

Das Auftreten von löslichen und unlöslichen Proteinen ergibt, wenn eine Rückfaltung bzw. Renaturierung der rMLA-Proteine möglich ist, mindestens zwei Verfahren zur Isolierung von rMLA. Das zu "inclusion bodies" aggregierte rMLA wurde nach Waschen der Sedimente zur Entfernung von *E. coli* Proteinen [Babbitt et al., 1990] unter denaturierenden Bedingungen gelöst und durch 90-fache Verdünnung in Faltungspuffer (50 mM Tris-HCl, 2 mM DTT, 1 mM EDTA, pH 8,0) rückgefaltet.

Es ergaben sich nach dieser Prozedur einerseits lösliche, gefaltete Proteinspezies, die wie in Fig. 9 dargestellt, ebenso wie die renaturierten, ursprünglich unlöslichen, denaturierten rMLA Spezies voll enzymatisch aktiv sind. Eine Isolierung der renaturierten rMLA kann wie auch die Isolierung der löslichen rMLA durch Immunaffinitätschromatographie unter Verwendung des spezifischen anti-MLA-Antikörpers TA5 erfolgen.

Mit dem Vorliegen von rMLB in löslicher Form als auch in Form unlöslicher "inclusion bodies" ergeben sich zwei Verfahren zur Isolierung von rekombinanter Mistellektin B-Kette.

Zur Isolierung der löslichen rMLB-Kette aus dem stark reduktiven Milieu des *E. coli* Cytoplasmas erfolgt zur Ausbildung der intrachenaren Disuldibrücken eine Inkubation in Anwesenheit eines Redox-Systems aus reduziertem und oxidiertem Glutathion sowie zur Stabilisierung aktiver Faltungsprodukte in Anwesenheit des Liganden β-Lactose. Aus dem Faltungsansatz wurde aktive, Carbohydrat-bindende rMLB-Kette durch Affinitätschromatographie an Lactosyl-Agarose in einem 1-Schritt-Verfahren selektiv isoliert bzw. gereinigt.

Zur Isolierung von rMLB aus dem unlöslichen, als "inclusion bodies" vorliegenden Expressionsproduktanteils wurde das Sediment des *E. coli* Gesamtzellaufschlußes zur Entfernung von *E. coli* Proteinen gewaschen [Babitt et al., 1990] und unter denaturierenden und reduzierenden Bedingungen gelöst. Die Renaturierung erfolgte durch Verdünnung in Anwesenheit eines Redox-Systems aus reduziertem und oxidiertem Glutathion sowie des Liganden β-Lactose, wobei aktive Carbohydrat-bindende rMLB-Kette durch Affinitätschromatographie an Lactosyl-Agarose selektiv aus dem Renaturierungsansatz isoliert und gereinigt werden.

Die Erfindung betrifft außerdem ein Arzneimittel, das das erfindungsgemäße Polypeptid und/oder das erfindungsgemäße Polypeptiddimer und/oder das nachstehend beschriebene erfindungsgemäße Immuntoxin, gegebenenfalls zusammen mit einem pharmazeutisch verträglichen Träger enthält.

Die erfindungsgemäßen Polypeptide, ihre Assoziate oder Modifikationen eignen sich für vielfältige Anwendungen in der Krebs- und Infektionstherapie, entsprechend den für das natürliche Mistellektin bekannten pharmakologischen Eigenschaften.
Die immunstimulierenden Wirkungen lassen sich für die Tumortherapie nutzen, indem durch direkte und/oder indirekte Stimulation die körpereigene Immunabwehr befähigt wird, den Tumor und eventuelle Metastasen wirksamer zu bekämpfen. Dasselbe gilt auch für Infektionen, insbesondere virale Erkrankungen.
Die erfindungsgemäßen Polypeptide können auch mit anderen Immunstimulantien, z.B. Interferonen, Cytokinen oder koloniestimulierenden Faktoren, in Kombination gegeben werden, um synergistische Wirkungen zu erzielen bzw. die notwendige Dosis der Kombinationspartner zu reduzieren und damit deren Nebenwirkungen zu vermindern.
In Kombination mit Cytostatika oder Bestrahlung läßt sich die Nebenwirkung der Leukopenie/Myelosuppression mildern oder vermindern, so daß die durch diese derzeit üblichen Behandlungsmethoden hervorgerufene Schwächung des Immunsystems reduziert wird.
Die direkte cytotoxische Wirkung der Polypeptide mit Glycosidaseaktivität führt zu Apoptose von Tumorzellen und kann ebenfalls zur Therapie genutzt werden. Dieses Prinzip kann bei der Verwendung von Immuntoxinen spezifischer gestaltet werden, wenn man die erfindungsgemäßen Polypeptide an geeignete Antikörper koppelt. Somit betrifft die Erfindung ferner Immuntoxine, die mindestens ein erfindungsgemäßes Polypeptid oder Polypeptiddimer umfassen. Beispielsweise kann eine Koppelung von aktiver A-Kette oder Holoprotein an Antikörper oder Fragmente hiervon mit proteinchemischen Methoden erfolgen. Solche Koppelungsverfahren sind dem Fachmann bekannt, die entsprechenden Konjugate vielseitig einsetzbar (Vitetta, 1993). Alternativ können auch entsprechend konstruierte Fusionsproteinkonstrukte zur Expression gebracht werden, die Antigen-bindende Domänen aus z.B. Antikörpern und zusätzlich cytotoxische Fragmente des erfindungsgemäßen Polypeptids enthalten.

Weiterhin läßt sich die Bildung von Metastasen verhindern, wenn die Bindung von Tumorzellen an andere Zellen inhibiert wird. Über kompetitive Lektinbindung kann mit dem erfindungsgemäßen Polypeptiden diese Bindung verhindert werden.

Schließlich betrifft die Erfindung diagnostische Zusammensetzungen, die mindestens enthalten:
a) das erfindungsgemäße Nukleinsäuremolekül;
b) einen Primer und/oder ein Primerpaar, der/das spezifisch an das erfindungsgemäße Nukleinsäuremolekül bzw. an den komplementären Strang dazu hybridisiert; und/oder
c) das erfindungsgemäße Polypeptid und/oder das erfindungsgemäße Polypeptiddimer.

Die erfindungsgemäße diagnostische Zusammensetzung kann in der Ausführungsform, die den Primer bzw. das Primerpaar enthält, dazu benutzt werden, Organismen nach der Existenz eines Lektingens abzusuchen, um so beispielsweise neue Lektingene aufzufinden, die gegebenenfalls pharmakologisch interessante Lektine codieren.

**Die Figuren zeigen**
- Fig. 1: Konstruktion der primären Amplifikationsoligonukleotide
- Fig. 2: Primäres *Viscum album* ML-Amplifikationsprodukt
- Fig. 3: Klonierstrategie zur Darstellung des ML-Gens
- Fig. 4: Insertionen der Expressionsvektoren für rMLA und rMLB und vollständige ML-Gensequenz
- Fig. 5: Konstruktionsschema Expressionsvektor für rMLA
- Fig. 6: Konstruktionsschema Expressionsvektor für rMLB
- Fig. 7: Expression von rMLA, rMLB und immunologische Detektion
- Fig. 8: IEF Chromatofocussierung von rMLA und rMLB vs. nat. ML
- Fig. 9: Enzymatische Aktivität von rMLA (RIP)
- Fig. 10: Carbohydrat-Bindecharakteristik von rMLB
- Fig. 11: Cytotoxizität des reassoziierten rMLA/MLB Holoproteins

### Beispiel 1

### Konstruktion der primären Amplifikationsoligonukleotide

Das Mistellektin (ML) gehört zur der Klasse der Ribosomeninaktivierenden Proteine [Stirpe et al., 1992), die eine in Pflanzen verschiedener taxonomischer Herkunft weit verbreitete Protein-Familie darstellt. ML wurde aufgrund der Aktivitäten seiner Unterheiten der Gruppe der Typ II Ribosomeninaktivierenden Proteinen zugeordnet [Endo et al., 1988a).

Zur Ermittlung der ML-Gensequenz ist der naheliegende Ansatz des Durchmusterns von *Viscum album* cDNA- und genomischen Genbanken jedoch ungeeignet. So konnten aus Genbanken aus *Viscum album* poly-A+ RNA trotz der Verwendung verschiedener DNA-Sonden keine ML-spezifischen Klone identifiziert werden. Mit der Annahme, daß die ML-Gensequenz keine Introns enthält, wurde deshalb eine PCR-Strategie angewandt. Da die N-terminalen Aminosäuresequenzen von der MLA- als auch der MLB-Kette bekannt waren [Dietrich et al., 1992; Gabius et al., 1992), erschien eine Amplifikation des MLA-codierenden Bereiches unter Verwendung von degenerierten, aus den bekannten Peptiden abgeleiteten Amplifikationsoligonukleotiden möglich (Fig. 1a). Während ein brauchbares Oligonukleotid mit geringem Degenerationsgrad aus dem N-Terminus der MLA-Kette abgeleitet werden kann (RMLA1, Fig. 1b), ist es nicht möglich, entsprechende Oligonukleotide mit ausreichender Spezifität aus dem N-Terminus der MLB-Kette zu konstuieren (RMLB1, RMLB2, RMLB3, Fig. 1b).

Es mußten deshalb alternative Strategien entwickelt werden, die durch das Einbeziehen von Proteindaten verwandter Proteine das Ableiten von Amplifikationsoligonukleotiden aus noch unbekannten ML-Sequenzbereichen ermöglichte. So zeigte eine Aminosäuresequenz-Analyse von Typ I und Typ II Ribosomen-inaktivierenden Proteinen eine Anzahl konservierter Bereiche mit hoher Sequenz-Homologie. Fig. 1c zeigt den hohen Verwandschaftsgrad von Typ I und Typ II RIP am Beispiel des aktiven Zentrums von Ricin. Innerhalb des Sequenzmotivs MISEAARF wurde für E177 und R180 die Beteiligung am enzymatischen Mechanismus diskutiert [Kim et al., 1992; Lord et al., 1994]. Daraus wurde geschlossen, daß zumindest diese beiden Reste in der ML-Sequenz vorhanden sein sollten. Aus weiteren strukturellen Überlegungen hinsichtlich dem Vorhandensein einzelner Reste, wobei besonderes solche mit einem niedrigen Degenerationsgrad des Codon-Gebrauchs berücksichtigt wurden, resultierte die Konstruktion des Amplifikationsoligonukleotids RMLA2. Die Sequenz dieses Oligonukleotids ist in Fig. 1c dargestellt.

### Beispiel 2

### Darstellung von ML-Gen spezifischen DNA-Fragmenten

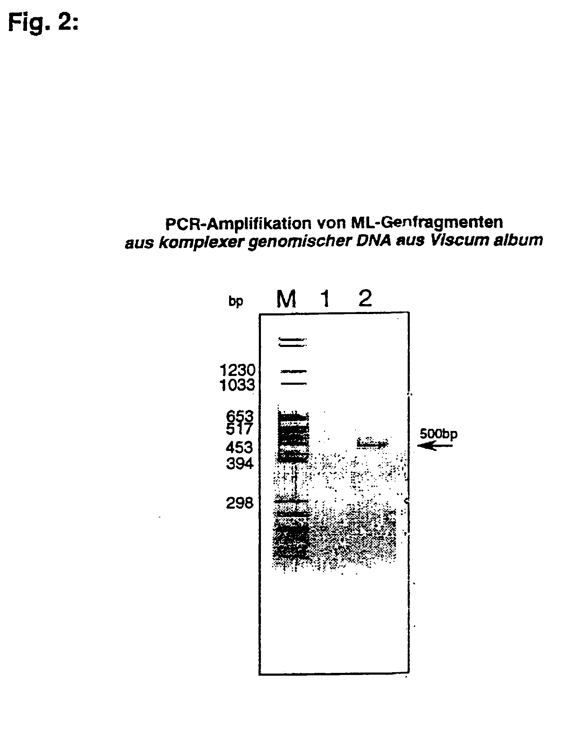

Hochmolekulare, genomische DNA wurde ausgehend von frischen *Viscum album* Blättern (Wirtsbaum *Populus wilsonii)* nach der Methode nach Baur et al. [1993] isoliert. Zur Darstellung von ML-Gen spezifischen DNA-Fragmenten durch PCR wurden je Amplifikationsansatz 100 ng genomische DNA eingesetzt. Die Amplifikation erfolgte in einem Gesamtvolumen von 50 µl, enthaltend PCR-Puffer (10 mM Tris-HCl, 1,5 mM MgCl₂, 50 mM KCl, 0,25 mM dNTP, pH 8,3 ), 78 pmol Primer RMLA1 und 50 pmol (Ansatz 2) bzw. 100 pmol (Ansatz 1) RMLA2. Die PCR erfolgte mit Taq-DNA-Polymerase (1,5E/Ansatz) von Boehringer Mannheim mit einem Biometra Thermocycler. Die PCR-Parameter waren: 1 min Denaturierung 90°C, 1 min Annealing 50°C, 1 min Elongation 72 °C bei insgesamt 30 Zyklen. Die Amplifikationsprodukte wurden durch 5%ige Polyacrylamidgel-Elektrophorese und Färbung mit Ethidiumbromid analysiert (Fig. 2) . Das in Ansatz 2 erhaltene spezifische Amplifikationsprodukt mit einer Größe von etwa 500 bp wurde durch Gelelution isoliert und der Klonierung in TA-Vektoren zugeführt.

### Beispiel 3

### Klonierungstrategie

Die Ableitung der zur primären PCR eingesetzten Amplifikationsoligonukleotide ist in Beispiel 1 (Fig. 1a), die Darstellung des primären Genfragments des *Viscum album* ML-Gens, im Folgenden als "a" bezeichnet, ist in Beispiel 2 (Fig. 2) gezeigt. Ausgehend von der Sequenz des klonierten Genfragments "a" und der Annahme, daß das ML-Gen keine Introns besitzt, war es nun möglich, sequenzspezifische 5'-Oligonukleotide abzuleiten, mit denen eine Amplifikation der Fragmente "b", "c", "d" und "e " ermöglicht wurde. Während das 3'-Oligonukleotid für "c" ebenfalls aus der DNA-Sequenz von "a" abgeleitet wurde, mußte die Konstruktion der degenerierten 3'-Primer zur Amplifikation der Genfragmente "b", "d", "e" und "g" durch Analyse homologer Bereiche von Typ I ("b") und Typ II ("d", "e", "g") RIP-Proteinen erfolgen. Hierbei wurde wiederum aus den Sequenzvergleichen innerhalb der Protein-Familien auf das Vorhandensein einzelner Reste geschlossen, wobei besonders Reste mit geringem Degenerationsgrad des Codon-Gebrauchs berücksichtigt wurden. Insbesondere die bekannten Ricin und Abrin cDNA- und abgeleitete Protein-Sequenzen wurden zur Konstruktion von etwa 50 ML-spezifischen Oligonukleotid-Kombinationen herangezogen. In Fig. 3 sind nur die Genfragmente gezeigt, die als spezifische Amplifikationsprodukte klonierbar waren und einer weiteren Analyse zugeführt werden konnten. Ausgehend von anderen Oligonukleotid-Kombinationen konnten keine ML-spezifischen Amplifikationsprodukte generiert werden. Die Darstellung der Genfragmente "f" (codierend für die MLA-Kette) und "g" (codierend für die MLB-Kette) ist im Detail in Beispiel 5 und Beispiel 6 beschrieben.

Zur Analyse der 5'- und 3'-Regionen der translatierten und nicht-translatierten Sequenzbereiche des ML-Gens wurden Bedigungen zur 5'- und 3'-RACE [Frohmann et al., 1988] etabliert, die zur Generierung der Fragmente "h" und "i" und "j" führte. Die Amplifikation des Fragments "j" mit RACE-PCR ist damit eine alternative zur Darstellung von vollständigen MLB-Genfragmenten. Die RACE-Reaktionen wurden unter Verwendung von cDNA durchgeführt, die durch reverse Transkription von isolierter *Viscum album* Gesamt-RNA aus Mistelblättern (Wirtsbaum *Populus wilsonii)* präpariert wurde.

### Beispiel 4

### DNA-Sequenz und Translationsprodukte rMLA und rMLB

Die Insertionen der Expressionsvektoren pT7-MLA und pT7-MLB wurden durch Standardverfahren mittels "primer walking" Strategie (Ermittlung vollständig überlappender Sequenzen beider Stränge) unter Verwendung verschiedener ML-spezifischer Oligonukleotide sequenziert (Fig. 4 a, b). Die unterstrichenen Sequenzbereiche bezeichnen Restriktionsschnittstellen zur Klonierung in die pT7-Expressionsvektoren. Beide Genfragmente sind entsprechend dem Konstruktionsschema der Expressionsvektoren wie in Beispiel 5 und Beispiel 6 beschrieben modifiziert.
Fig. 4c zeigt die aus den bezeichneten Fragmenten abgeleitete vollständige ML-Gensequenz. Sie umfaßt auch 5_- und 3_-nichttranslatierte Bereiche sowie Endopeptid- und Signalpeptid-codierende Abschnitte.

### Beispiel 5

### Konstruktion des Expressionsvektors pT7-MLA

Zur heterologen Expression wurde die für die Mistellektin A-Kette codierende Sequenz durch spezifische PCR ausgehend von komplexer genomischer Mistel-DNA dargestellt und terminal modifiziert. Über nicht-komplementäre Bereiche der eingesetzten Primer-Oligonukleotide wurden hierbei Translations-Kontrollelemente sowie Erkennungssequenzen von Restriktionsendonukleasen angefügt, wodurch ausgehend von dem genomisch vorliegenden Präpromistellektin-Gen die Klonierung und getrennte Expression der Mistellektin A-Kette ermöglicht wurde.

Fig. 5b zeigt die Darstellung von MLA-codierenden Gen-Fragmenten durch PCR. Zur Amplifikation der MLA-codierenden Gensequenz wurden 200 ng genomische *Viscum album* DNA, 1,5 mM (Ansatz 1) bzw. 2,5 mM (Ansatz 2) Magnesiumchlorid, 40 pmol von jedem Primer Oligonukleotid RML16 und RML17 in PCR-Puffer (10 mM Tris-HCl, 50 mM KCl, 0,25 mM von jedem dNTP, pH 8,3 ) in einem Gesamtvolumen von 50 µl eingesetzt. Die PCR erfolgte unter Verwendung von Taq-Polymerase (1,5U/Ansatz, Boehringer Mannheim) durch insgesamt 30 Zyklen des Temperaturprofils 1 min Denaturierung 94 °C, 1 min Annealing 52 °C, 1,5 min Elongation 72 °C. Die Amplifikationsprodukte wurden durch 1%ige Agarosegel-Elektrophorese und Färbung mit Ethidiumbromid analysiert (Fig. 5b) und durch Gelelution der Klonierung in TA-Vektoren zugeführt.

Der 5'-Bereich der für rMLA codierenden Sequenz entsprechend der Aminosäurereste Tyrosin¹ - Tyrosin¹⁷ [Dietrich et al., 1992; Gabius et al., 1992] wurde unter Vorschaltung eines Translations-Startcodons als synthetisches Genfragment durch Hybridisierung und Klonierung zweier Oligonukleotide dargestellt. Hierdurch wurde eine Optimierung der Gensequenz hinsichtlich der Codon-Auswahl, wie sie für stark exprimierte Gene in *Escherichia coli* beschrieben ist [Gribskov et al., 1984], erzielt. Am 3'-Ende des synthetischen rMLA-Genfragments sowie am 5'-Ende des mittels PCR dargestellten rMLA-Genfragments wurde durch den gezielten Austausch des Tyrosin¹⁷-Codons von TAC zu TAT eine *Ssp I* - Restriktionsschnittstelle eingeführt, die eine Fusion der beiden rMLA-Genfragmente unter Darstellung des Vektors pML14-17 (Beispiel 5, Fig. 5) ermöglichte.

Die generierte vollständige für rMLA codierende Sequenz wurde durch DNA-Sequenzierung bestätigt (Fig. 4a). Zur Expression von rMLA in *Escherichia coli* wurde die Gensequenz aus dem Vektor pML14-17 isoliert und durch Insertion in den Expressionsvektor pT7-7 unter die Kontrolle des T7-RNA Polymerase Promotors sowie Transkriptionsterminators gestellt. Mit dem resultierenden Expressionsvektor pT7-MLA (Fig. 5) wurde der *E. coli* Expressionsstamm BL21 transformiert.

### Beispiel 6

### Konstruktion des Expressionsvektors pT7-MLB

Zur heterologen Expression der Mistellektin B-Kette wurde die vollständige, MLB codierende Sequenz durch spezifische PCR aus komplexer genomischer *Viscum album* DNA amplifiziert, wobei über nicht-komplementäre Bereiche der eingesetzten Primer-Oligonukleotide Translationskontrollelemente sowie Erkennungssequenzen für Restriktionsendonukleasen eingeführt wurden.

Fig. 6b zeigt die Darstellung des gesamten, rMLB vollständig codierenden Genfragments durch PCR. Die Amplifikation des rMLB-codierenden DNA-Fragments erfolgte ausgehend von jeweils 200 ng genomischer *Viscum album* DNA in PCR-Ansätzen mit 50 pmol Primer-Oligonukleotid RML25 und 30 pmol (Ansatz 1) bzw. 10 pmol (Ansatz 2) Primer-Oligonukleotid RML26 in einem Gesamtvolumen von 50 µl PCR-Puffer (10 mM Tris-HCl, 50 mM KCl, 1,5 mM MgCl₂, 0,25 mM von jedem dNTP, pH 8,3). Die PCR erfolgte unter Verwendung von Taq-Polymerase (1,5 E/Ansatz, Boehringer Mannheim) durch 30 Zyklen aus 1 min Denaturierung 94 °C, 1 min Annealing 52 °C und 1,5 min Elongation 72 °C. Die PCR-Produkte wurden durch 1%ige Agarosegel-Elektrophorese und Färbung mit Ethidiumbromid analysiert. Es resultierte ein 0,8 kbp PCR-Produkt, das durch Gelelution isoliert und der Klonierung in TA-Vektoren zugeführt wurde. Durch Insertion in den Expressionsvektor pT7-7 wurde das rMLB-codierende Genfragment unter die Kontrolle von Transkriptionskontrollelementen gestellt und mit dem resultierenden Expressionsvektor pT7-MLB der Expressionsstamm *E. coli* BL21 transformiert. Die Integrität der PCR-generierten, vollständigen, für rMLB-codierenden Sequenz wurde durch DNA-Sequenzierung bestätigt (Fig. 4b).

### Beispiel 7

### Expression, immunologischer Nachweis, Rückfaltung und in vitro Reassoziation von rMLA und rMLB

### (I) Expression von rMLA in E. coli

Zur Expression von rekombinanter Mistellektin A-Kette wurden 1000 ml LB_{Amp}-Medium in 2 Ltr. Schüttelkolben mit Schikanen mit 5 ml einer stationär gewachsenen LB_{Amp}-Vorkultur von *E. coli* BL21/pT7-MLA angeimpft und bei 37 °C unter Schütteln kultiviert, wobei das Wachstum durch Trübungsmessung bei 578 nm verfolgt wurde. Die Genexpression wurde bei Erreichen einer Zelldichte entsprechend OD₅₇₈ Å 0,9 - 1,0 durch die Zugabe von 0,5 mM IPTG induziert. Zur Ernte wurden die Zellen 2 h nach Induktion durch Zentrifugation 20 min bei 5.000 rpm und 4 °C im GS-3 Rotor (Sorvall) sedimentiert und das Kulturmedium dekantiert, wobei ausgehend von 1 Ltr. Kulturvolumen eine Zellmasse von 3 - 4 g (Feuchtgewicht) isoliert wurde.

Der Zellaufschluß erfolgte mittels French-Press (SLM Instruments), wozu das Zellsediment in 20 ml Aufschlußpuffer (50 mM Tris-HCl, 100 mM NaCl, 1 mM EDTA, 5 mM DTT, 1 mM PMSF, pH 8,0) resuspendiert und durch 2 French-Press Durchgänge bei 1.500 psi aufgeschlossen wurde. Durch eine anschließende Zentrifugation für 30 min bei 10.000 UpM und 4 °C im SS-34 Rotor (Sorvall) wurden unlösliche Zellbestandteile sowie enthaltene "inclusion bodies" sedimenditert und von den im Überstand verbleibenden löslichen *E. coli* Proteinen bzw. löslichen Expressionsprodukten getrennt.

Zur Analyse der Expression wurden gleiche Volumina der Zellaufschlußfraktionen durch 12,5 % SDS-Polyacrylamidgel-Elektrophorese und Coomassie-Brilliant-Blau Färbung sowie durch Western-Blot unter Verwendung des MLA-spezifischen Antiserums TA5 untersucht (Fig. 7a). Hierzu wurden gleiche Volumen der löslichen Fraktion (Spur 2, 4, 6, 8) sowie der unlöslichen "inclusion bodies" Fraktion (Spur 1, 3, 5, 7) des *E. coli* Aufschlußes hinsichtlich des Gehaltes an rMLA untersucht. Zur Darstellung des Expressionsverlaufes wurden dabei Proben vor (Spur 1+2), 2 h (Spur 3+4), 4 h (Spur 5+6) und 6 h (Spur 7-8) nach Induktion der Genexpression eingesetzt. Die Expression ist schon 1h nach Induktion durch das Auftreten eines immunreaktiven 25 kDa Expressionsproduktes entsprechend rMLA gekennzeichnet, deren Expressionsmaximum bereits 2 h nach Induktion erreicht ist. Die Verteilung von rMLA auf die lösliche bzw. unlösliche Zellaufschlußfraktion beträgt 2. h nach Induktion jeweils ^{~}50 %, wobei eine längere Expressionsdauer zu einer zunehmenden Bildung unlöslicher "inclusion bodies" führt.

### (II) Isolierung von rMLA aus unlöslichen "inclusion bodies"

Das Sediment des *E. coli* Gesamtzellaufschlußes wurde zur Entfernung von *E. coli* Proteinen 2x mit je 20 ml STET-Puffer (50 mM Tris-HCl, 8 % (w/v) Sucrose, 50 mM EDTA, 0,05 % (v/v) Tween-20, pH 7,4) nach Babitt et al. [1990] gewaschen. Das verbleibende Sediment mit den enthaltenen "inclusion bodies" wurde in 20 ml Denaturierungspuffer (6 M Guanidiniumchlorid, 100 mM DTT, 50 mM Tris-HCl, pH 8,0) durch Inkubation 16 h bei Raumtemperatur unter Schütteln gelöst.

Zur Renaturierung von rMLA wurde die im Denaturierungspuffer vorliegende Proteinlösung in das 90-fache Volumen Faltungspuffer (50 mM Tris-HCl, 2 mM DTT, 1 mM EDTA, pH 8,0) langsam eingetropft und 16 h bei Raumtemperatur unter Rühren inkubiert. Wieder ausgefallenes Protein wurde durch Zentrifugation 30 min bei 6.000 rpm und 4 °C im GS-3 Rotor (Sorvall) abgetrennt. Der rMLA-haltige Überstand wurde zur Lagerung auf 20 % (v/v) Glycerin eingestellt und bei 4 °C aufbewahrt.

### (III) Reinigung von rMLA durch Immunaffinitätschromatographie

Zur 1-Schritt-Reinigung von rMLA (löslicher Expressionsproduktanteil bzw. rückgefaltetes Protein) durch Immunaffinitätschromatographie wurden 260 µg des gegen Mistellektin A-Kette gerichteten, monoklonalen Antikörpers anti-nMLA-IgG (TA5) an Protein-A-Sepharose CL4B (Sigma, Deisenhofen) kovalent nach der Methode von Harlow & Spur [1988] immobilisiert. Nach Inkubation der Immunaffinitätsmatrix mit der rMLA-Probe und Waschen der Matrix mit 10 Säulenbettvolumen Waschpuffer 1 (1 M NaCl,10 mM Phosphat-Puffer, pH 7,0) und 10 Säulenbettvolumen Waschpuffer 2 (10 mM Phosphat-Puffer, pH 7,0) zur Entfernung unspezifisch gebundener Proteine wurde spezifisch gebundene rMLA mit Elutionspuffer (0,1 M Glycin, pH 2,5) eluiert. Die Elution erfolgte zur Rückstellung des pH-Wertes in eine Vorlage von 1 M Phosphat-Puffer, pH 8,0.

### (IV) Expression von rMLB in E. coli

Zur Expression von rekombinanter Mistellektin B-Kette wurden 1000 ml LB_{Amp}-Medium in 2 Ltr. Schüttelkolben mit Schikanen mit 5 ml einer stationär gewachsenen LB_{Amp}-Vorkultur von *E. coli* BL21/pT7-MLB angeimpft und bei 37 °C unter Schütteln kultiviert, wobei das Wachstum durch Trübungsmessung bei 578 nm verfolgt wurde. Die Genexpression wurde bei Erreichen einer Zelldichte entsprechend OD₅₇₈ Å 0,9 - 1,0 durch die Zugabe von 0,5 mM IPTG induziert. Zur Ernte wurden die Zellen 4 h nach Induktion durch Zentrifugation 20 min bei 5.000 UpM und 4 °C im GS-3 Rotor (Sorvall) sedimentiert und das Kulturmedium dekantiert.

Der Zellaufschluß erfolgte mittels French-Press™ (SLM Instruments), wozu das Zellsediment in 20 ml Aufschlußpuffer B (20 mM Phosphat-Puffer, 50 mM NaCl, 1 mM EDTA, 1 mM PMSF, pH 7,2) resuspendiert und durch 2 French-Press Durchgänge bei 1.500 psi aufgeschlossen wurde. Durch eine anschließende Zentrifugation für 30 min bei 10.000 UpM und 4 °C im SS-34 Rotor (Sorvall) wurden unlösliche Zellbestandteile sowie enthaltene "inclusion bodies" sedimendiert und von den im Überstand verbleibenden löslichen *E. coli* Proteinen bzw. löslichem Expressionsprodukt getrennt.

Zum Nachweis der Expression wurden gleiche Volumina der Zellaufschlußfraktionen durch 12,5 % SDS-Polyacrylamidgel-Elektrophorese und Coomassie-Brilliant-Blau Färbung sowie durch Western-Blot unter Verwendung des MLB-spezifischen Antiserums TB33 untersucht (Fig. 7b). Hierzu wurden gleiche Volumen der löslichen Fraktion (Spur 2, 4,6, 8) sowie der unlöslichen "inclusion bodies" Fraktion (Spur 1, 3, 5, 7) des *E. coli* Aufschlußes hinsichtlich des Gehaltes an rMLB untersucht. Zur Darstellung des Expressionsverlaufes wurden dabei Proben vor (Spur 1+2), 2 h (Spur 3+4), 4 h (Spur 5+6) und 6 h (Spur 7-8) nach Induktion der Genexpression eingesetzt. Die Western-Blot Anlyse zeigte bereits 1 h nach Induktion das Auftreten einer immunreaktiven 31 kDa Proteinbande entsprechend rMLB, wobei das Expressionsmaximum 4 h nach Induktion erreicht wurde. 4 h nach Induktion verteilt sich die rMLB-Menge zu jeweils ^{~} 50% auf die lösliche bzw. unlösliche Fraktion des Zellaufschlußes. Längere Inkubationszeiträume führen zu einer zunehmenden Akkumulation exprimierter rMLB in Form unlöslicher "inclusion bodies".

### (V) Isolierung von rMLB aus unlöslichen "inclusion bodies"

Das Sediment des *E. coli* Gesamtzellaufschlußes wurde zur Entfernung von *E. coli* Proteinen 2x mit je 20 ml STET-Puffer (50 mM Tris-HCl, 8 % (w/v) Sucrose, 50 mM EDTA, 0,05 % (v/v) Tween-20, pH 7,4) nach Babbitt et al. [1990] gewaschen. Das verbleibende Sediment mit den enthaltenen "inclusion bodies" wurde in 20 ml Denaturierungspuffer (6 M Guanidiniumchlorid, 100 mM DTT, 50 mM Tris-HCl, pH 8,0) durch Inkubation 16 h bei Raumtemperatur unter Schütteln gelöst.

Zur Renaturierung von rMLB wurde die im Denaturierungspuffer vorliegende Proteinlösung in das 90-fache Volumen Faltungspuffer (20 mM Phosphat-Puffer, 50 mM NaCl, 1 mM EDTA, 5 mM Glutathion reduziert, 1 mM Glutathion oxidiert, 10 mM β-Lactose, pH 7,2) langsam eingetropft und 16 h bei Raumtemperatur unter Rühren inkubiert. Wieder ausgefallenes Protein wurde durch Zentrifugation 30 min bei 6.000 UpM und 4 °C im GS-3 Rotor (Sorvall) von der löslichen, gefalteten rMLB-Fraktion abgetrennt.

### (VI) Isolierung von rMLB durch Affinitätschromatographie an Lactosyl-Agarose

Zur Isolierung von aktiver, Carbohydrat-bindender rMLB wurde eine Lactosyl-Agarose Affinitätsmatrix (Sigma, Deisenhofen) mit 10 Säulenbettvolumen Chromatographiepuffer (20 mM Phosphat-Puffer, 50 mM NaCl, 1 mM EDTA, pH 7,2) äquilibiert. Der Probenauftrag erfolgte durch "batch" Inkubation der Affinitätsmatrix in rMLB-haltigen Probenlösung für mind. 2 h bei Raumtemperatur. Nach Waschen der Affinitätsmatrix mit Chromatographiepuffer zum Entfernen unspezifisch gebundener Proteine wurde gebundenes rMLB durch kompetitive Verdrängung mit 0,3 M β-Lactose in Chromatographiepuffer eluiert.

### (VII) In vitro Reassoziation von Mistellektin-Ketten zur Darstellung des Holoproteins

Zur Darstellung von rekombinantem Mistellektin-Holoprotein wurde isolierte Mistellektin B-Kette mit einem molaren Überschuß an rMLA in 20 mM Phosphat-Puffer, 50 mM NaCl, 1 mM EDTA, pH 7,2 für 16 - 48 h bei Raumtemperatur inkubiert. Zur Ausbildung der interchenaren Disulfidbrücke erfolgte die Inkubation in Anwesenheit eines Redox-Systems aus 6 mM Glutathion (Verhältnis reduziert zu oxidiert 5 : 1) oder 10 mM Glutathion (Verhältnis reduziert zu oxidiert 2 : 1) + 1 µM Protein-Disulfid-Isomerase (Boehringer, Mannheim). Nachweis und Identifizierung des gebildeten Heterodimers erfolgte duch SDS-PAGE unter nicht-reduzierenden Bedingungen und anschließende Western-Blot Analyse unter Verwendung spezifischer, monoklonaler Antikörper gegen Mistellektin A-Kette (TA5) bzw. Mistellektin B-Kette (TB33). Die Isolierung des gebildeten Holoproteins bzw. Abtrennung freier rMLA bzw. rMLA-Aggregaten erfolgte durch Affinitätschromatographie an Lactosyl-Agarose wie unter (VI) beschrieben.

### Beispiel 8

### Isoelektrische Homogenität von rMLA und rMLB

1-2 µg rMLA, natürliches MLA, rMLB, natürliches MLB oder ML-Holoprotein wurden zusammen mit IEF-Proteinstandards (BioRad, USA) auf Servalyt PreNets IEF-Gelen (pH 3 -10, 125 x 125 mm, 150 µm , Serva Heidelberg) fokussiert. Zum Nachweis wurden die Proteine durch "semi-dry electroblotting" auf Nitrocellulose-Membranen (0,2 µm, Schleicher & Schüll, Dassel) immobilisiert. Die immunologische Färbung erfolgte mit Hilfe eines monoklonalen, MLA-spezifischen Antiserums (TA5 aus Maus) für rMLA und nMLA bzw. mit Hilfe eines monoklonalen, MLB-spezifischen Antiserums (TB33 aus Maus) für rMLB, nMLB und ML-Holoprotein. Immunkomplexe wurden unter Verwendung eines mit alkalischer Phosphatase konjugierten anti-Maus IgG-IgG (Sigma, Deisenhofen) und Umsetzung der Substrate NBT und BCIP angefärbt (Fig. 8).

Diskussion der Ergebnisse erfolgt in der obigen Beschreibung unter "Untersuchung der Eigenschaften von rMLA und rMLB, Punkt (II) Isoelektrische Homogenität.

### Beispiel 9

### Nachweis der enzymatischen, Ribosomen-inaktivierenden Aktivität von rMLA

Die Proteinkonzentration der mittels Immunaffinitätschromatographie gereinigten rMLA (refolded) und rMLA (soluble) sowie von natürlicher MLA-Kette (nMLA) wurde nach Bradford [1976] unter Verwendung eines BSA-Standards bestimmt.

Zum Nachweis und Quantifizierung der enzymatischen rRNA-N-Glycosidase-Aktivität von MLA wurde unter Verwendung des "TNT coupled reticulocyte system" (Promega, USA) ein nicht-radioaktives Testsystem etabliert. Je Ansatz wurden gleiche Mengen (20 µl) des TNT-Systems 15 min bei 30 °C vorinkubiert. Zur Quantifizierung einer Translationsinhibiton wurden den Kontrollansätzen 2 µl des entsprechenden Puffers bzw. den Testansätzen 2 µl steigender MLA-Verdünnungen (Konzentrationsbereich 350 - 0 pM) zugesetzt. Aus jedem Ansatz wurden im Abstand von 8 min 2 Proben entnommen und zum Abstoppen der Reaktion in flüssigem Stickstoff eingefroren. Als Maß für die Translations-Aktivität wurde die relative Luciferase-Menge (sqrt-cpm) durch einen Bioluminiszens-Test mittels Szintillationsmeßgerät bestimmt. Für jeden Ansatz wurde hierbei die Differenz der gemessenen sqrt-cpm der beiden Zeitproben als Maß der relativen Translations-Aktivität ermittelt, wobei die Aktivität im Kontrollansatz ohne RIP auf 0 % Inaktivierungsrate (IAR) gesetzt wurde.

Durch Auftragung der relativen Translations-Inaktivierungsrate gegen die eingesetzten rMLA-Konzentrationen wurde mittels nichtlinearer Regression diejenige Proteinkonzentration ermittet, die zu 50 % Inhibition der Translationsaktivität im Vergleich zum Kontrollansatz führt. Dieser IC₅₀-Wert stellt eine System-abhängige Größe dar, die einen Nachweis und Quantifizierung der enzymatischen Aktivität von rMLA (soluble), rMLA (refolded) im Vergleich zu nMLA ermöglicht (Fig. 9).

Fig. 9 zeigt den Nachweis der enzymatischen, Ribosomen-inaktivierenden Aktivität der rekombinanten MLA-Kette, wobei sowohl durch Isolierung des löslichen Expressionsproduktanteils (rMLA soluble) als auch durch Rückfaltung des aus "inclusion bodies" isolierten Proteins (rMLA refolded) enzymatisch aktives Expressionsprodukt erhalten wird. rMLA (soluble) und rMLA (refolded) weisen mit IC₅₀-Werten von 10,7 ± 1,3 pM bzw. 15,6 ± 6,6 pM übereinstimmende Aktivitäten auf. Sie weisen damit eine niedrigere toxische Aktivität auf als die natürliche MLA-Kette (IC₅₀ 1,1 ± 0,7 pM).

### Beispiel 10

### Carbohydrat-bindende Aktivität der Mistellektin B-Kette

100 µl einer Lösung von 1,1 mg Asialofetuin (Sigma, Deisenhofen) in 11 ml PBS wird in die Kavitäten von MaxiSorp C96 Mikrotiterplatten (Nunc, Wiesbaden) gegeben und zur Beschichtung mit der galaktosyl-haltigen Schicht 16 h bei Raumtemperatur inkubiert. Nach Waschen der Mikrotiterplatten 3 x mit 150 µl / Kavität PBS-T ( 10 mM Natriumphosphat-Puffer, 130 mM NaCl, 0,1 % (v/v) Tween 20, pH 7,2) wurde die Mikrotiterplatte zum Blockieren unspezifischer Bindungsstellen mit 200 µl / Kavität PBS-T-1% BSA (10 mM Natriumphosphat-Puffer, 130 mM NaCl, 0,1 % (v/v) Tween 20, 1 % (w/v) BSA, pH 7,2) für 1 h bei 36 °C inkubiert und anschließend wieder wie beschrieben gewaschen. Zum Test wurden 100 µl B-Ketten haltiger Präparationen oder ML-1 Referenz lösungen im Konzentrationsbereich von 10 - 200 ng/ml eingesetzt, wobei die entsprechenden Verdünnungsreihen in PBS-0,05% BSA (10 mM Natriumphosphat-Puffer, 130 mM NaCl, 0,05 % (w/v) BSA, pH 7,2) erstellt wurden. Pro Probenkonzentration und Kontrolle wurden 2-3 Replikas angelegt. Die Bestimmung des Leerwertes erfolgt mit PBS-0,05% BSA oder dem jeweilen Präparationspuffer. Die Platten wurden nach Beladung 2 h bei 36 °C inkubiert und anschließend wie beschrieben gewaschen. In die belegten Kavitäten wurde 100 µl / Kavität anti-Mistellektin-Serum aus Ziege (1:19800 Verdünnung des Serumpools in PBS-T-0,1% BSA-Tx (10 mM Natriumphosphat-Puffer, 130 mM NaCl, 0,1 % (v/v) Tween 20, 0,1 % (w/v) BSA, 1 % (v/v) Triton X100, pH 7,2)) gegeben, 2 h bei 36 °C inkubiert und anschließend wie beschrieben gewaschen. Zum Nachweis der Immunkomplexe wurde je belegte Kavität 100 µl anti-Ziege IgG-IgG, konjugiert mit Meerrettich-Peroxidase (Sigma, Deisenhofen) in einer 1 : 3500 Verdünnung in PBS-1% BSA (10 mM Natriumphosphat-Puffer, 130 mM NaCl, 1 % (w/v) BSA, pH 7,2) zugegeben und 1 h bei 36 °C inkubiert. Die Kavitäten wurden anschließend 6x mit 150 µl/Kavität PBS-T gewaschen. Zur Entwicklung wurden 100 µl / Kavität Substratlösung (1 o-Phenylendiamin-Tablette (Sigma, Deisenhofen) in 25 ml 65 mM Citronensäure, pH 5,0 + 10 µl 30 % Wasserstoffperoxid) zugegeben und 20 min bei Raumtemperatur im Dunkeln inkubiert. Das Abstoppen der Reaktion erfolgte durch die Zugabe von 100 µl / Kavität 1 M Schwefelsäure. Die Auswertung erfolgte durch Absorptionsmessung bei 450 nm mit einer Referenzwellenlänge von 690 nm.

Fig. 10 zeigt eine Auftragung der im ELLA-System beobachteten Verdrängung von rMLB und nML Lektinen vom immobilisierten Asialofetuinliganden durch steigende Mengen D-Galactose. Die Bindecharakteristiken von MLB und rMLB, ausgedrückt durch den IC50 Wert bei halbmaximaler Verdrängung durch Galactose von nML 1,65 mM und rMLB 1,3 mM unterscheiden sich.

### Beispiel 11

### Bestimmung der Cytotoxizität in vitro reassoziierter rML-Holoproteine auf humane lymphatische Leukämiezellen

Die Integrität des Mistellektin-Holoproteins wurde durch die quantitative Bestimmung der Cytotoxizität gegenüber der humanen lymphatischen Leukämiezellinie MOLT-4 (European Collection Of Animal Cell Cultures No. 85011413) nachgewiesen.

MOLT-4 Zellen wurden in serumfreiem MDC-1 Medium (PAN SYSTEMS, Aidenbach) kultiviert und für den Test auf eine Einsaatzelldichte von 1,6 x10⁵ MOLT-4-Zellen/ml bei einer Viabilität > 98 % eingestellt. Zur Bestimmung der Cytotoxizität wurden pro Kavität einer 96-Loch Mikrotiterplatte 90 µl einer MOLT-4 Zellsuspension entsprechend 15000 Zellen/Kavität eingesät und mit 10 µl der Probenlösung versetzt. Zum Test wurde Mistellektin-Holoprotein Präparationen (Chargen 220793 und BR1294) sowie in vitro reassoziiertes rMLA/MLB Holoprotein im Konzentrationsbereich 0,05 - 200 pg/ml entsprechend 0,8 fM - 3,32 pM eingesetzt, wobei die entsprechenden Verdünnungsreihen in MDC-1 Zellkulturmedium erstellt wurden. Pro Probenkonzentration und Kontrolle wurden 6 Replikas angelegt.
Die Zellen wurden 72 h bei 37 °C und 5 % CO₂ im begasten Brutschrank inkubiert. Die Quantifizierung des cytotoxischen Effektes erfolgte durch die Bestimmung der Viabilität der Zellen mit Hilfe eines löslichen Formazan-Farbstoffes nach der XTT-Methode oder WST-1 Methode [Scudiero et al., 1988] unter Verwendung des entsprechenden Cell Proliferation Kit II XTT (Boehringer, Mannheim) bzw. Cell Proliferation Reagent WST-1 (Boehringer Mannheim).

Diskussion der Ergebnisse erfolgt in der obigen Beschreibung unter "Untersuchung der Eigenschaften von rMLA und rMLB, Punkt (V) Cytotoxizität in vitro assoziierter ML-Holoproteine.

### Literatur

Babbitt, P.C., West, B.L., Buechter, D.D., Kuntz, I.D. und Kenyon, G.L. [1990]: Removal of a proteolytic activity associated with aggregates formed from expression of creatine kinase in *Escherichia coli* leads to improved recovery of active enzyme. Bio/Technology **8**, 945-949.

Baur, A., Buschinger, A. und Zimmermann, F.K. (1993): Molecular cloning and sequencing of 18S rDNA fragments of six different ant species. Ins. Soc. **40**, 325-335.

Beuth, J., Ko, H.L., Gabius, H.-J. und Pulverer, G. [1991]: Influence of treatment with the immunomodulatory effective dose of the β-galactoside-specific lectin from Mistletoe on tumor colonization in BALB/c-mice for two experimental model systems. in vivo **5**, 29-32.

Beuth, J., Ko, H.L., Gabius, H.-J., Burrichter, H., Oette, K. und Pulverer, G. [1992]: Behavior of lymphocyte subsets and expression of activation markers in response to immunotherapy with galactoside-specific lectin from mistletoe in breast cancer patients. Clin. Investig. **70**, 658-661.

Beuth, J., Ko, H.L., Tunggal, L., Steuer, M.K., Geisel, J., Jeljaszewicz, J. und Pulverer, G. [1993]: Thymocyte proliferation and maturation in response to galactoside-specific Mistletoe Lectin-1. in vivo **7**, 407-410.

Beuth, J., Ko, K.L., Tunggal, L., Geisel, J. und Pulverer, G. [1993]: Vergleichende Untersuchungen zur immunaktiven Wirkung von Galaktosid-spezifischem Mistellektin. Arzneim. Forsch. **43**, 166-169.

Bocci, V. [1993]: Mistletoe (*Viscum album*) lectins as cytokine inducers and immunoadjuvant in tumor therapy. J. of Biological Regulators and Homeostatic Agents **7**, 1-6.

Beuth, J., Ko, H.L., Tungal, L., Buss, G., Jeljaszewicz,J., Steuer, M.K. und Pulverer, G. [1994]: Immunaktive Wirkung von Mistellektin-1 in Abhängigkeit von der Dosierung. Arzneim. Forschung 44, 1255-1258.

Bradford, M.M. [1976]: A rapid and sensitive method for the quantitation of microgram quantities of protein utilizing the principle of protein-dye binding. Analytical Biochemistry **72**, 248-254.

Dietrich, J.B., Ribéreau-Gayon. G., Jung, M.L., Franz, H., Beck, J.P. und Anton, R. [1992]: Identity of the N-terminal sequences of the three A chains of mistletoe (*Viscum album* L.) lectins: homology with ricin-like plant toxins and single-chain ribosome-inhibiting proteins. Anti-Cancer Drugs **3**, 507-511.

Eifler, R., Pfüller, K., Göckeritz, W. und Püller, U. [1994]: Improved procedures for isolation and standardization of mistletoe lectins and their subunits: implications for the analysis of lectin pattern of the european mistletoe. In: Lectins Biology-Biochemistry Clinical Biochemistry (Bog-Hansen, Hrsg.) Vol. 9, M/S Wiley, New Delhi.

Endo, Y., Tsurugi, K. und Franz, H. [1988a]: The site of action of the A-chain of mistletoe lectin I on eukaryotic ribosomes. The RNA N-glycosidase activity of the protein. FEBS Lett. **231**, 378-80.

Endo, Y., Tsurugi, K. und Lambert, J.M. [1988b]: The site of action of six differtent ribosom-inactivating proteins from plants on eukaryotic ribosomes: the RNA N-glycosidase activity of the proteins. Biochem. Biophys. Res. Commun. **150**, 1032-1036.

Erlich, H.A., Gelfand, D.H. und Saiki, R.K. [1988]: Specific DNA amplification. Nature **331**, 461.

Franz, H., Haustein, B., Luther, P., Kuropka, U. und Kindt, A. [1977]: Isolierung und Charakterisierung von Inhaltsstoffen der Mistel (Viscum album L.) I. Affinitätschromatographie an fixierten Plasmaproteinen. Acta biol. med. germ. **36**, 113-117.

Frohman, M.A., Dush, M.K. und Martin, G.R. [1988]: Rapid production of full-length cDNAs from rare transcripts: Amplification using a single gene-specific oligonukleotide primer. Proc. Natl. Acad. Sci. USA **85**, 8998-9002.

Gabius, H.-J., Walzel, H., Joshi, S.S., Kruip, J., Kojima, S., Gerke, V., Kratzin, H. and Gabius, S. [1992]: The immunomodulatory β-galactoside-specific lectin from Mistletoe: Partial sequence analysis, cell and tissue binding, and impact on intracellular biosignalling of monocytic leukemia cells. Anticancer Res. **12**, 669-676.

Gabius, H.-J., Gabius, S., Joshi, S.S., Koch, B., Schroeder, M., Manzke, W.M. und Westerhausen, M. [1993]: From III-defined extracts to the immunomodulatory lectin: Will there be a reason for oncological application of Mistletoe ? Planta Med. **60**, 2-7.

Gabius, H.-J. und Gabius, S. [1994]: Die Misteltherapie auf dem naturwissenschaftlichen Prüfstand. PZ **139**, 9-16.

Ganguly, C. und Das, S. [1994]: Plant Lectins as inhibitors of tumor growth and modulators of host immune response. Chemotherapy **40**, 272-278.

Garcia-Olmedo, F., Carbonero, P., Hernandez-Lucas, C., Paz-Ares, J., Ponz, F., Vicente, O. und Sierra, J.M. [1983]: Inhibition of eukaryotic cell-free protein synthesis by thionins from Wheat endosperm. Biochim. Biophys. Acta **740**, 52-56.

Gribskov, M., Devereux, J. und Burgess, R. [1984] Nucl. Acids Res. **12**, 539-549.

Hajto, T. [1986]: Immunmodulatory effects of Iscador: A Viscum album preparation. Oncology 43 suppl.1, 51-65.

Hajto, T., Hostanska, K. und Gabius, H.-J. [1989]: Modulatory potency of the β-galactoside-specific lectin from Mistletoe extract (Iscador) on the host defense system in vivo in Rabbits and Patients. Cancer Res. **49**, 4803-4808.

Hajto, T., Hostanska, K., Frei, K., Rordorf, C. und Gabius, H.-J. [1990]: Increased secretion of Tumor necrosis factor a, Interleukin 1, and Interleukin 6 by Human Mononuclear Cells exposed to β-Galactoside-specific lectin from clinically applied Mistletoe extract. Cancer Res. **50**, 3322-3326.

Harlowe, E. und Spur, D. [1988] In: Antibodies. A laboratory manual. Cold Spring Harbor Laboratory, Cold Spring Harbor, New York.

Heiny, B.-M. und Beuth, J. [1994]: Mistletoe extract standardized for the galactoside-specific lectin (ML-1) induces β-endorphin release and immunopotentiation in breast cancer patients. Anticancer Research **14**, 1339-1342.

Hülsen, H., Doser, C. und Mechelke, F. [1986]: Differences in the in vitro effectiveness of preparations produced from Mistletoes of various host trees. Drug. Res. **36**, 433-436.

Janssen, O., Scheffler, A., und Kabelitz, D. [1993] In vitro Effects of Misteletoe Extracts and Mistletoe Lectins. Cytotoxicity towards tumor cells due to the induction of programmed cell death (apoptosis). Arzneim. forsch. 43, 1221-1227

Katzin, B.J., Collins, E.J. und Robertus, J.D. [1991]: Structure of Ricin A-Chain at 2.5 Å. Proteins **10**, 251-259.

Kim, Y., Misna, D., Monzingo, A.F., Ready, M.P., Frankel, A., und Robertus, J.D. [1992]: Structure of a Ricin mutant showing rescue of activity by a noncatalytic residue. Biochemistry, **31**, 3294-3296.

Lord, J.M., Roberts, L.M. und Robertus, J.D. [1994]: Ricin: structure, mode of action, and some current applications. FASEB J. **8**, 201-208.

Männel, D.N., Becker, H., Gundt, A., Kist, A. und Franz, H. [1991]: Induction of tumor necrosis factor expression by a lectin from Viscum album. Cancer Immunol. Immunother. **33**, 177-182.

Minowada, J., Ohnuma, T., Moore, G.E. [1972] Rosette-forming human lymphoid cell lines. J. Nat. Cancer Inst. 49, 891-895

Mendez, E., Moreno, A., Colilla, F., Pelaez, F., Limas, G.G., Mendez, R., Soriano, F., Salinas, M. und de Haro, C. [1990]: Primary structure and inhibition of protein synthesis in eukaryotic cell-free system of a novel thionin, g-hordothionin, from barley endosperm. Eur. J. Biochem. **194**, 533-539.

Rutenber, E. und Robertus, J.D. [1991]: Structure of Ricin B-chain at 2.5 Å resolution. Proteins **10**, 260-269.

Scudiero, P.A. et al. [1988] Cancer Res. **48**, 4827-4823.

Stein, G. und Berg, P.A. [1994]: Non-lectin component in a fermented extract from Viscum album L. grown on pines induces proliferation of lymphocytes from heallthy and allergic individuals in vitro. Eur. J. Clin. Pharmacol. **47**, 33-38.

Stirpe, F., Barbieri, L., Battelli, M.G., Soria, M. und Lappi, D.A. [1992]: Ribosome-inactivating proteins from plants: Present status and future prospects. Bio/Technology **10**, 405-412.

Studier, F.W. und Moffart, B.A. [1986]: Use of Bacteriophage T7 RNA Polymerase to direct selective high-level expression of cloned genes. J. Mol. Biol. **189**, 113-130.

Vitetta, E. S., Thorpe, P. E., Uhr, J. W. (1993) Immunotoxins: magic bullets or misguided missiles? Immunol. Today, 14, 252-259

Weston, S.A., Tucker, A.D., Thatcher, D.R., Derbyshire, D.J. und Pauptit, R.A. [1994]: X-ray structure of recombinant Ricin A-Chain at 1.8 Å resolution. J. Mol. Biol. 244, 410-422.

## Patentansprüche

1. Nukleinsäuremolekül, das
(a) ein Präproprotein codiert, das nach Reifung die biologische Funktion des Mistellektindimers aufweist und die in Fig. 4c dargestellte Nukleotidsequenz aufweist;
(b) ein Fragment des Präproproteins gemäß (a) codiert, wobei das Fragment natürlicherweise ein biologisch aktiver Bestandteil des Mistellectindimers ist;
(c) sich durch die Degeneration des genetischen Codes vom Nukleinsäuremolekül gemäß (a) oder (b) unterscheidet; oder
(d) unter stringenten Bedingungen mit dem Nukleinsäuremolekül gemäß (a), (b) oder (c) hybridisiert und ein Polypeptid mit der in (a) oder (b) angegebenen biologischen Funktion codiert.

2. Nukleinsäuremolekül nach Anspruch 1, wobei das Fragment die A-Kette des Mistellektins ist, die durch die in Fig. 4a dargestellte Nukleotidsequenz codiert wird.

3. Nukleinsäuremolekül nach Anspruch 1, wobei das Fragment die B-Kette des Mistellektins ist, die durch die in Fig. 4b dargestellte Nukleotidsequenz codiert wird.

4. Nukleinsäuremolekül nach einem der Ansprüche 1 bis 3, das ein DNA-Molekül ist.

5. Nukleinsäuremolekül nach einem der Ansprüche 1 bis 3, das ein RNA-Molekül ist.

6. Vektor, der mindestens ein Nukleinsäuremolekül nach einem der Ansprüche 1 bis 4 enthält.

7. Vektor nach Anspruch 6, der sowohl ein Nukleinsäuremolekül nach Anspruch 2 oder 4, sowie auch ein Nukleinsäuremolekül nach Anspruch 3 oder 4 enthält.

8. Vektor nach Anspruch 6 oder 7, der ein Expressionsvektor ist.

9. Wirt, der mit mindestens einem Vektor nach einem der Ansprüche 6 bis 8 transformiert ist, wobei der transformierte Wirt kein Mensch, keine transgene Tierart, keine transgene Pflanze und keine transgene Pflanzensorte ist.

10. Wirt nach Anspruch 9, der eine Pflanzenzelle ist.

11. Wirt nach Anspruch 9, der eine Säugerzelle, ein Bakterium, eine Pilzzelle, eine Hefezelle oder eine Insektenzelle ist.

12. Wirt nach Anspruch 11, wobei das Bakterium E. coli ist, die Pilzzelle eine Aspergillus-Zelle und die Insektenzelle eine Spodoptera-Zelle, vorzugsweise eine Spodoptera frugiperda-Zelle ist.

13. Polypeptid, das von einem Nukleinsäuremolekül nach einem der Ansprüche 1 bis 5 kodiert und vom Wirt nach einem der Ansprüche 11 oder 12 produziert wird, oder das von einem Vektor nach einem der Ansprüche 6 bis 8 kodiert und vom Wirt nach einem der Ansprüche 11 oder 12 produziert wird, oder das vom Wirt nach einem der Ansprüche 11 oder 12 produziert wird.

14. Polypeptid nach Anspruch 13, das mindestens eine chemische oder enzymatische Modifizierung aufweist, wobei die enzymatische Modifizierung keine in Viscum album vorkommende Glykosylierung ist.

15. Polypeptid nach Anspruch 13 oder 14, das ein Fusionsprotein ist.

16. Polypeptiddimer mit der biologischen Funktion des Mistellektins, wobei das eine Monomere vom Nukleinsäuremolekül nach einem der Ansprüche 2, 4 oder 5 und das zweite Monomere vom Nukleinsäuremolekül nach einem der Ansprüche 3 bis 5 codiert wird und mindestens eines der Monomere vom Wirt nach Anspruch 11 oder 12 produziert wird.

17. Polypeptiddimer mit der biologischen Funktion des Mistellektins, wobei das eine Monomere vom Nukleinsäuremolekül nach einem der Ansprüche 2, 4 oder 5 und das zweite Monomere vom Nukleinsäuremolekül nach einem der Ansprüche 3 bis 5 codiert wird, wobei mindestens eines der Monomere ein Polypeptid nach Anspruch 14 oder 15 ist.

18. Verfahren zur Herstellung des Polypeptids nach Anspruch 13 oder des Polypeptiddimers nach Anspruch 16, wobei man den Wirt nach einem der Ansprüche 9 bis 12 unter geeigneten Bedingungen züchtet und das so erhaltene Polypeptid oder Polypeptiddimer isoliert.

19. Immuntoxin, umfassend mindestens ein Polypeptid nach einem der Ansprüche 13 bis 15 oder ein Polypeptiddimer nach Anspruch 16 oder 17.

20. Arzneimittel, enthaltend das Polypeptid nach einem der Ansprüche 13 bis 15 und/oder das Polypeptiddimer nach Anspruch 16 oder 17 und/oder das Immuntoxin nach Anspruch 19, gegebenenfalls zusammen mit einem pharmazeutisch verträglichen Träger.

21. Diagnostische Zusammensetzung mindestens enthaltend
(a) das Nukleinsäuremolekül nach einem der Ansprüche 1 bis 5;
(b) einen Primer und/oder ein Primerpaar, der/das spezifisch an das Nukleinsäuremolekül gemäß (a) bzw. an den komplementären Strang dazu hybridisiert; und/oder
(c) das Polypeptid nach einem der Ansprüche 13 bis 15 und/oder das Polypeptiddimer nach Anspruch 16 oder 17.

## Claims

1. A nucleic acid molecule
(a) encoding a preproprotein that exhibits after maturation the biological function of the mistletoe lectin dimer and which has the nucleic acid sequence as depicted in Fig. 4c;
(b) encoding a fragment of a preproprotein according to (a), with the fragment naturally being a biologically active part of the mistletoe lectin dimer;
(c) differing from the nucleic acid molecule according to (a) or (b) due to the degeneracy of the genetic code; or
(d) hybridizing under stringent conditions to the nucleic acid molecule according to (a), (b) or (c) and encoding a polypeptide having the biological function indicated in (a) or (b).

2. The nucleic acid molecule according to claim 1, wherein the fragment is the A chain of the mistletoe lectin which is coded for by the nucleotide sequence depicted in Fig. 4a.

3. The nucleic acid molecule according to claim 1, wherein the fragment is the B chain of the mistletoe lectin which is coded for by the nucleotide sequence depicted in Fig. 4b.

4. The nucleic acid molecule according to any of claims 1 to 3 which is a DNA molecule.

5. The nucleic acid molecule according to any of claims 1 to 3 which is an RNA molecule.

6. A vector which contains at least one nucleic acid molecule according to any of claims 1 to 4.

7. The vector according to claim 6 which contains both a nucleic acid molecule according to claim 2 or 4 and a nucleic acid molecule according to claim 3 or 4.

8. The vector according to claim 6 or 7 which is an expression vector.

9. A host which is transformed with at least one vector according to any of claims 6 to 8, wherein said transformed host is no human, no transgenic animal species, no transgenic plant and no transgenic plant variety.

10. The host according to claim 9, which is a plant cell.

11. The host according to claim 9, which is a mammalian cell, a bacterium, a fungal cell, a yeast cell or an insect cell.

12. The host according to claim 11, wherein the bacterium is E. coli, the fungal cell is an Aspergillus cell and the insect cell is a Spodoptera cell, preferably a Spodoptera frugiperda cell.

13. A polypeptide which is coded for by the nucleic acid molecule according to any of claims 1 to 5 and produced by the host according to claim 11 or 12 or which is coded for by the vector according to any of claims 6 to 8 and produced by the host according to claim 11 or 12 or which is produced by the host according to claim 11 or 12.

14. The polypeptide according to claim 13 which exhibits at least one chemical or enzymatic modification, wherein the enzymatic modification is not a glycosylation occuring in Viscum album.

15. The polypeptide according to claim 13 or 14 which is a fusion protein.

16. A polypeptide dimer having the biological function of the mistletoe lectin, wherein one monomer is coded for by the nucleic acid molecule according to any of claims 2, 4 or 5 and the second monomer by the nucleic acid molecule according to any of claims 3 to 5 and wherein at least one of the monomers is produced by the host according to claim 11 or 12.

17. A polypeptide dimer having the biological function of the mistletoe lectin, wherein one monomer is coded for by the nucleic acid molecule according to any of claims 2, 4 or 5 and the second monomer by the nucleic acid molecule according to any of claims 3 to 5 and wherein at least one of the monomers is a polypeptide according to claim 14 or 15.

18. A process for producing the polypeptide according to claim 13 or the polypeptide dimer according to claim 16 wherein the host according to any of claims 9 to 12 is cultured under appropriate conditions and the polypeptide or polypeptide dimer so obtained is isolated.

19. An immunotoxin comprising at least one polypeptide according to any of claim 13 to 15 or a polypeptide dimer according to claim 16 or 17.

20. A pharmaceutical composition comprising the polypeptide according to any of claims 13 to 15 and/or the polypeptide dimer according to claim 16 or 17 and/or the immunotoxin according to claim 19, optionally in admixture with a pharmaceutically acceptable carrier.

21. A diagnostic composition containing at least
(a) the nucleic acid molecule according to any of claims 1 to 5,
(b) a primer and/or a primer pair specifically hybridyzing to the nucleic acid molecule of (a) or a complementary strand thereof and/or
(c) the polypeptide according to any of claims 13 to 15 and/or the polypeptide dimer according to claim 16 or 17.

## Revendications

1. Molécule d'acide nucléique, qui
(a) code une préprotéine, qui après maturation présente la fonction biologique de la lectine dimère du gui, et présente la séquence nucléotidique représentée sur la Figure 4c ;
(b) code un fragment de la préprotéine selon (a), le fragment étant naturellement un constituant biologiquement actif de la lectine dimère du gui ;
(c) se distingue de la molécule d'acide nucléique selon (a) ou (b) par la dégénérescence du code génétique ; ou bien
(d) s'hybride dans des conditions stringentes avec la molécule d'acide nucléique selon (a), (b) ou (c), et code un polypeptide ayant la fonction biologique indiquée en (a) ou (b).

2. Molécule d'acide nucléique selon la revendication 1, dans laquelle le fragment est la chaîne A de la lectine du gui, qui est codée par la séquence nucléotidique représentée sur la Figure 4a.

3. Molécule d'acide nucléique selon la revendication 1, dans laquelle le fragment est la chaîne B de la lectine du gui, qui est codée par la séquence nucléotidique représentée sur la Figure 4b.

4. Molécule d'acide nucléique selon l'une des revendications 1 à 3, qui est une molécule d'ADN.

5. Molécule d'acide nucléique selon l'une des revendications 1 à 3, qui est une molécule d'ARN.

6. Vecteur qui contient au moins une molécule d'acide nucléique selon l'une des revendications 1 à 4.

7. Vecteur selon la revendication 6, qui contient non seulement une molécule d'acide nucléique selon la revendication 2 ou 4 mais aussi une molécule d'acide nucléique selon la revendication 3 ou 4.

8. Vecteur selon la revendication 6 ou 7, qui est un vecteur d'expression.

9. Hôte, qui est transformé avec au moins un vecteur selon l'une des revendications 6 à 8, l'hôte transformé n'étant ni un homme, ni une espèce animale transgénique, ni une plante transgénique, ni une variété végétale transgénique.

10. Hôte selon la revendication 9, qui est une cellule végétale.

11. Hôte selon la revendication 9, qui est une cellule de mammifère, une bactérie, une cellule fongique, une cellule de levure ou une cellule d'insecte.

12. Hôte selon la revendication 11, dans lequel la bactérie est E. coli, la cellule fongique est une cellule d'Aspergillus et la cellule d'insecte est une cellule de spodoptère, de préférence une cellule de Spodoptera frugiperda.

13. Polypeptide, qui est codé par une molécule d'acide nucléique selon l'une des revendications 1 à 5 et est produit par l'hôte selon l'une des revendications 11 ou 12, ou qui est codé par un vecteur selon l'une des revendications 6 à 8 et est produit par l'hôte selon l'une des revendications 11 ou 12, ou encore qui est produit par l'hôte selon l'une des revendications 11 ou 12.

14. Polypeptide selon la revendication 13, qui comporte au moins une modification chimique ou enzymatique, la modification enzymatique n'étant pas une glycosylation se produisant dans Viscum album.

15. Polypeptide selon la revendication 13 ou 14, qui est une protéine de fusion.

16. Polypeptide dimère ayant la fonction biologique de la lectine du gui, où l'un des monomères est codé par la molécule d'acide nucléique selon l'une des revendications 2, 4 ou 5 et le deuxième monomère est codé par la molécule d'acide nucléique selon l'une des revendications 3 à 5, au moins l'un des monomères étant produit par l'hôte selon la revendication 11 ou 12.

17. Polypeptide dimère ayant la fonction biologique de la lectine du gui, où l'un des monomères est codé par la molécule d'acide nucléique selon l'une des revendications 2, 4 ou 5 et le deuxième monomère est codé par la molécule d'acide nucléique selon l'une des revendications 3 à 5, au moins l'un des monomères étant un polypeptide selon la revendication 14 ou 15.

18. Procédé pour préparer le polypeptide selon la revendication 13 ou le polypeptide dimère selon la revendication 16, dans lequel on sélectionne l'hôte selon l'une des revendications 9 à 12 dans des conditions appropriées, et on isole le polypeptide ou le polypeptide dimère ainsi obtenu.

19. Immunotoxine, comportant au moins un polypeptide selon l'une des revendications 13 à 15 ou un polypeptide dimère selon la revendication 16 ou 17.

20. Médicament, contenant le polypeptide selon l'une des revendications 13 à 15 et/ou le polypeptide dimère selon la revendication 16 ou 17 et/ou l'immunotoxine selon la revendication 19, éventuellement en même temps qu'un excipient compatible d'un point de vue pharmaceutique.

21. Composition diagnostique, contenant au moins :
(a) la molécule d'acide nucléique selon l'une des revendications 1 à 5 ;
(b) une amorce et/ou une paire d'amorces, qui s'hybride d'une manière spécifique à la molécule d'acide nucléique selon (a) ou au brin complémentaire de cette dernière ; et/ou
(c) le polypeptide selon l'une des revendications 13 à 15 et/ou le polypeptide dimère selon la revendication 16 ou 17.
